(19) Europäisches Patentamt
European Patent Office
Office européen des brevets

(11) **EP 4 417 179 A1**

(12) **EUROPÄISCHE PATENTANMELDUNG**

(43) Veröffentlichungstag:
**21.08.2024 Patentblatt 2024/34**

(21) Anmeldenummer: 24155689.3

(22) Anmeldetag: **05.02.2024**

(51) Internationale Patentklassifikation (IPC):
*A61K 6/17* (2020.01)    *A61K 6/76* (2020.01)
*A61K 6/77* (2020.01)    *A61K 6/887* (2020.01)
*B33Y 70/10* (2020.01)    *B33Y 80/00* (2015.01)

(52) Gemeinsame Patentklassifikation (CPC):
(C-Sets verfügbar)
**A61K 6/887; A61K 6/17; A61K 6/76; A61K 6/77;
B33Y 70/10; B33Y 80/00**      (Forts.)

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(30) Priorität: **16.02.2023 DE 102023103800**

(71) Anmelder: **VOCO GmbH
27472 Cuxhaven (DE)**

(72) Erfinder:
• **Oldenburger, Daniel
27476 Cuxhaven (DE)**
• **Metzscher, Sarah
27628 Hagen i.Br. (DE)**
• **Plaumann, Manfred Thomas
27472 Cuxhaven (DE)**

(54) **RADIKALISCH POLYMERISIERBARE ZUSAMMENSETZUNG ZUM 3D-DRUCK VON DENTALEN KRONEN, BRÜCKEN, PROTHESENZÄHNEN ODER VOLLPROTHESEN**

(57) Die vorliegende Erfindung betrifft radikalisch polymerisierbare Zusammensetzungen zum 3D-Druck von dentalen Kronen, Brücken, Prothesenzähnen oder Vollprothesen, die sich aufgrund ihrer Füllstoffkombination durch sehr gute mechanische Eigenschaften und eine optimale Sedimentationsstabilität auszeichnen.

EP 4 417 179 A1

(52) Gemeinsame Patentklassifikation (CPC): (Forts.)

C-Sets
**A61K 6/887, C08L 33/08;**
**A61K 6/887, C08L 33/10**

**Beschreibung**

Die vorliegende Erfindung betrifft radikalisch polymerisierbare Zusammensetzungen zum 3D-Druck von dentalen Kronen, Brücken, Prothesenzähnen oder Vollprothesen.

Die Erfindung wird in den beigefügten Patentansprüchen definiert. Bevorzugte Aspekte der vorliegenden Erfindung ergeben sich überdies aus der nachfolgenden Beschreibung einschließlich der Beispiele.

Soweit für einen erfindungsgemäßen Aspekt (Zusammensetzung, gehärtetes Produkt, therapeutische Anwendung) bestimmte Ausgestaltungen als bevorzugt bezeichnet werden, gelten die entsprechenden Ausführungen jeweils auch für die anderen Aspekte der vorliegenden Erfindung, *mutatis mutandis.* Bevorzugte individuelle Merkmale erfindungsgemäßer Aspekte (wie in den Ansprüchen definiert und/oder in der Beschreibung offenbart) sind miteinander kombinierbar und werden vorzugsweise miteinander kombiniert, sofern sich im Einzelfall für den Fachmann aus dem vorliegenden Text nichts anderes ergibt.

Für die Herstellung von Zahnrestaurationen, wie Kronen, Brücken, Prothesenzähnen oder Vollprothesen werden heutzutage noch überwiegend konventionelle Verfahren genutzt, die mehrere zeitintensive Schritte umfassen. Häufig wird auch ein subtraktives CAD/CAM-Verfahren zur Herstellung von Zahnrestaurationen angewendet. Hierbei wird aus einem vorgefertigtem CAD/CAM-Block die gewünschte Restauration herausgefräst. Auch diese Methode ist aufwendig und es entsteht ein großer Materialverlust beim Herausfräsen der Zahnrestauration, wodurch die Kosten dementsprechend hoch ausfallen.

In den letzten Jahren gewinnen generative Fertigungsverfahren, wie z.B. die Stereolithographie, in der Zahnmedizin zunehmend an Bedeutung, so auch für die Herstellung von Zahnrestaurationen.

In der Stereolithographie werden geeignete Harzsysteme mit einem ultravioletten Laser schichtweise ausgehärtet. Im Allgemeinen werden klassische (Meth)acrylatharze, die mit geeigneten Photoinitiatoren versehen sind, polymerisiert. Eine vertikal bewegliche Plattform mit einer geeigneten Oberfläche (Metall, Glas, Keramik, etc.) zum Anwachsen der festen Harzphase wird auf den Abstand einer Schichtdicke (20 - 200 $\mu$m), gemessen vom Grund des flüssigen Harzbades, abgesenkt. Es erfolgt nun eine lokal punktuelle Vernetzung und Aushärtung des Harzes durch einen Laser, der beispielsweise von unten mithilfe von Mikrospiegeln ins Harzbad geführt wird. Das feste Polymerisat, das sich auf der Plattform bildet, wird nach der Vernetzung einer ersten Harzschicht um eine weitere Schichtdicke im Harzbad hochbewegt und somit erneut mit flüssigem Harz belegt. Der Laser härtet jetzt die zweite Schicht. Die Reihenfolge der Anhebung der mit Harzschichten bestückten beweglichen Plattform und das darauffolgende Polymerisieren werden so lange wiederholt, bis das gesamte dreidimensionale Werkstück aufgebaut ist.

Stereolithographische Verfahren haben unter anderem den Vorteil, dass diese weniger zeitintensiv und genauer sind als die konventionellen Herstellungsverfahren von Kronen, Brücken, Prothesenzähnen oder Vollprothesen. Es können auch mehrere Restaurationen in einem Durchlauf hergestellt werden. Im Vergleich zum subtraktiven CAD/CAM-Verfahren fällt der Materialverlust bei der Stereolithographie viel geringer aus, da das nicht ausgehärtete Material wiederverwendet werden kann.

Für die Stereolithographie ist es meist von Vorteil, wenn das genutzte Harz eine geringe Viskosität hat. In der Regel erreicht man dies indem man einen geringen Füllstoffanteil einsetzt. Die Folge eines geringen Füllstoffanteils ist jedoch, dass die erforderlichen mechanischen Eigenschaften, wie zum Beispiel Festigkeit und Abrasionsresistenz oft nicht den Anforderungen für Zahnrestaurationen genügen.

Es ist also notwendig für die Herstellung von Zahnrestaurationen mittels Stereolithographie, eine Zusammensetzung bereit zu stellen, die sowohl im unpolymerisierten Zustand, als auch nach der Polymerisation als fertiges Produkt den jeweiligen Anforderungen entspricht.

Zusammensetzung zum 3D-Druck von Zahnrestaurationen sind aus dem Stand der Technik bekannt.

Die WO 2014/078537 A1 (Dentsply) offenbart eine Zusammensetzung für die Herstellung von dentalen Produkten mittels 3D-Druck, umfassend (Meth)acrylat, anorganischen Füllstoff, organischen Füllstoff, "silico-acrylic-based rubber impact modifier", Pigmente und Photoinitiatoren.

Die WO 2017/223084 A1 (Dentsply) beansprucht ein durch 3D-Druck hergestelltes dentales Produkt (z.B. Zahnprothese, Prothesenbasis), welches aus mindestens zwei Schichten besteht, die unterschiedliche Zusammensetzungen umfassen. Die erste Schicht umfasst ein Oligomer, eine polymerisierbare Acrylverbindung, "a rubber impact modifier" und einen Photoinitiator. Die zweite Schicht enthält ein Urethanmonomer, ein GlykolDimethacrylat, einen Füllstoff und einen Photoinitiator.

Die WO 2019/048963 A1 (3M) beschreibt eine druckbare Zusammensetzung mit einer Viskosität von weniger als 150 Pa*s mit mindestens einer durch Strahlung härtbaren Komponente, einen Photoinitiator und 50 Gew.-% eines Füllstoffs mit einer mittleren Partikelgröße von 0,3 $\mu$m. Die Zusammensetzung ist geeignet um Zahnrestaurationen, zum Beispiel Kronen und Brücken, herzustellen.

In WO 2017/155692 A1 (3M) wird eine vorgefertigte Zahnkrone beansprucht, die durch ihre Form und ihre chemische Zusammensetzung charakterisiert wird. Die Zusammensetzung umfasst 20 bis 70 Gew.-% Nanofüllstoffe und 20 bis 75 Gew.-% Harzmatrix, die Urethan(meth)acrylate und (Meth)acrylate ohne Urethaneinheit, enthalten.

Die WO 2015/126862 A1 (3M) offenbart eine Dentalzusammensetzung, die ein bestimmtes unsymmetrisches polymerisierbares Monomer, Initiator(en) und Füllstoff(e) in einer Menge von mehr als 20 Gew.-% enthält. Die Zusammensetzung ist geeignet zur Herstellung von Füllungsmaterial, Kronen, Brücken, Inlays, Onlays, Veneers und Fräsrohlingen.

In der WO 2015/126666 A1 (3M) wird eine Dentalzusammensetzung beschrieben, umfassend ein bestimmtes unsymmetrisches polymerisierbares Monomer, Initiator und Füllstoffkomponente(n) in einer Menge von mehr als 20 Gew.-%. Die Zusammensetzung ist geeignet für die Herstellung Füllungsmaterial, Zahnzement, Kronen, Brücken, Inlays, Onlays, Veneers, kieferorthopädische Vorrichtungen und Fräsrohlingen.

In der WO 2018/231583 A1 (3M) wird eine Zusammensetzung zur Herstellung von Zahnkronen beansprucht mit einer Viskosität von weniger als 150 Pa*s und einer Scherrate von 1 s$^{-1}$. Die beanspruchte Zusammensetzung umfasst polymerisierbare(s) (Meth)acrylat(e) ohne Urethankomponente, polymerisierbare(s) Urethan(meth)acrylat(e), eine Füllstoffmatrix in der Menge von 5 bis 45 Gew.-% mit Nanocluster(n) und optional pyrogene Kieselsäure in der Menge von weniger als 8 Gew.-%, ein Initiatorensystem mit Photoiniator(en) und organische(n) Farbstoff(e). Weiterhin enthält die beanspruchte Zusammensetzung keinen Weichmacher in einer Menge von mehr als 5 Gew.-%.

Die EP 3 795 359 A1 (Shofu) offenbart eine Zusammensetzung zur Herstellung einer Zahnrestauration mittels 3D-Druck. Die Zusammensetzung umfasst ein polymerisierbares Monomer auf (Meth)acrylatbasis, bestehend aus einem polymerisierbaren Monomer auf (Meth)acrylatbasis, das eine Urethanstruktur enthält, und einem polymerisierbaren Monomer auf (Meth)acrylatbasis, das keine Urethanstruktur enthält, einen kohäsiven anorganischen Füllstoff und einen Photoinitiator mit bestimmten Verhältnissen zueinander.

Die EP 1 508 834 A1 (3D Systems) beansprucht eine Zusammensetzung, die für die Herstellung dreidimensionaler Gegenstände durch Stereolithographie geeignet ist. Diese Zusammensetzung umfasst mindestens eine radikalisch polymerisierende organische Substanz, mindestens einen Initiator für die radikalische Polymerisation, mindestens einen Füllstoff, der Nanopartikel vom Siliciumdioxidtyp umfasst, mindestens eine kationisch polymerisierende organische Substanz, mindestens einen kationischen Polymerisationsinitiator, gegebenenfalls mindestens eine hydroxylfunktionelle Verbindung und gegebenenfalls mindestens eine Art von Mikropartikelfüllstoff.

Die WO 2014/098956 A1 (Dentca) beschreibt eine Zusammensetzung zur Herstellung von künstlichen Zähnen und Prothesen, die difunktionelles Bisphenol-A-Dimethacrylat, multifunktionelles Methacrylat, Urethandimethacrylat, oberflächenmodifizierte feine Teilchen auf Siliziumdioxidbasis, einen Photopolymerisationsinitiator, ein Farbmittel und mindestens eine Art von Stabilisator umfasst.

Die WO 2001/012679 A1 (Deltamed) beansprucht eine Zusammensetzung, die folgende Bestandteile enthält: 2 bis 99 Gew.-% mindestens einer Verbindung, die mindestens eine Acrylatgruppe und/oder mindestens eine Methacrylatgruppe und/oder mindestens eine Vinylgruppe und/oder mindestens eine Epoxidgruppe und/oder mindestens eine Oxetangruppe und/oder mindestens eine Acryl-Epoxy-Oligomergruppe und/oder mindestens eine Methacryl-Epoxy-Oligomergruppe enthält, und/oder mindestens einer Harzmasse auf der Basis mindestens eines polymerisierbaren Polysiloxans, 0,01 bis 7 Gew.-% mindestens eines Initiators, 0 bis 5 Gew.-% mindestens eines Coinitiatiors, 0 bis 85 Gew.-% eines oder mehrerer Modifikatoren, wie Füllstoffen, Farbstoffen, Pigmenten, Fließverbesserern, Thixotropiemitteln, polymeren Verdickern, oxidierend wirkenden Zusatzstoffen, Stabilisatoren und Verzögerern. Dies Zusammensetzung ist unter anderem geeignet zur Herstellung von Inlays, Onlays, Zahnfüllungen, Attachments, Kronen, Brücken, künstlichen Zähnen, Stiftzähnen, Zahnprothesen und Zahnimplantaten.

Die EP 3 669 854 A1 (GC) offenbart eine Zusammensetzung, die unter anderem für die Herstellung von Inlays, Kronen, Brücken und Prothesen geeignet ist. Die Zusammensetzung umfasst eine (Meth)acrylatverbindung, einen Füllstoff, einen Photopolymerisationsinitiator und ein Phthalsäurederivat und/oder ein Thiophenderivat, wobei der Gehalt des Phthalsäurederivats und/oder des Thiophenderivats größer als oder gleich 0,05 Gew.-% und kleiner als oder gleich 0,25 Gew.-% ist.

Die Zusammensetzung (mit dynamischer Viskosität) der WO 2020/177921 A1 (Mühlbauer) enthält radikalisch photopolymerisierbare Monomere und/oder Oligomere, einen in die in der Zusammensetzung eingearbeiteten organisch oberflächenmodifizierten und gegebenenfalls teilweise agglomerierten und/oder aggregierten nanoskaligen Füllstoff, mindestens einen Photoinitiator, optional einen Stabilisator, optional Pigmentpartikel und optional stabilisiertes Radikal zur stereolithographischen Herstellung eines dentalen Formteils, insbesondere Brücken und Kronen.

In der EP 3 479 171 A1 (3M) wird ein Verfahren zur Herstellung eines dreidimensionalen Gegenstands beschrieben. Das Verfahren umfasst die Bereitstellung einer druckbaren Zusammensetzung, die eine hochviskose polymerisierbare Komponente, ein temporäres Lösungsmittel und einen Initiator umfasst, das selektive Aushärten der druckbaren Zusammensetzung, um einen Gegenstand zu bilden, der die Form des dreidimensionalen Objekts darstellt, das Entfernen einer wesentlichen Menge des temporären Lösungsmittels aus dem Gegenstand und optionales Härten der unpolymerisierten polymerisierbaren Komponente, die vor oder nach dem Entfernen einer wesentlichen Menge des temporären Lösungsmittels verbleibt.

Die WO 2020/016282 A1 (Fraunhofer-Gesellschaft zur Förderung der angewandten Forschung) beansprucht ein Verfahren zum Herstellen eines Formkörpers mit Hilfe eines strahlungsinduzierten Druckverfahrens, sowie ein Formkörper auf Basis eines organisch polymerisierten Kieselsäure(hetero)polykondensats, welches durch organische Polymerisation einer Polysiloxan-Komponente erzeugt wurde. Der Formkörper ist durch einen E-Modul von mindestens 1,4 GPa gekennzeichnet und ist geeignet zur Verwendung als temporärer oder dauerhafter Zahnersatz.

Obwohl aus dem Stand der Technik einige Zusammensetzungen bekannt sind, die für den 3D-Druck von dentalen Restaurationen eingesetzt werden können, besteht im Rahmen der fortschreitenden digitalen Zahnheilkunde ein zunehmender Bedarf an Materialien, die in ihren mechanischen Eigenschaften den herkömmlichen Materialen vergleichbar sind. Gerade in punkto Sedimentationsstabilität besteht ein erheblicher Verbesserungsbedarf. In der Regel müssen die bisherigen Druckharze vor Gebrauch aufgeschüttelt werden. Mit zunehmender Standzeit in der Harz-Wanne des 3D-Druckers erfolgt aber eine erneute Sedimentation und die Produktqualität nimmt ab oder das Druckharz muss verworfen und ausgetauscht werden.

Aufgabe der Erfindung war es daher Zusammensetzungen bereitzustellen, die einerseits über gute mechanische Eigenschaften wie hohe Festigkeit und hohe Abrasionsstabilität verfügen. Andererseits müssen die Zusammensetzungen eine niedrige Viskosität aufweisen, um zuverlässig in 3D-Druckverfahren (SLA, DLP) eingesetzt werden zu können und dort eine generative Fertigung von dentalen Restaurationen mit hoher Genauigkeit zu gewährleisten. Zudem sollen die Zusammensetzungen über ein hohes Maß an Sedimentationsstabilität verfügen, um während des gesamten 3D-Druckprozesses gleichbleibend gute Produkte herstellen zu können.

Gelöst wird die Aufgabe durch eine radikalisch polymerisierbare Zusammensetzung zum 3D-Druck von dentalen Kronen, Brücken, Prothesenzähnen oder Vollprothesen umfassend

A) eine Gesamtmenge an radikalisch polymerisierbaren (Meth)acryl-Verbindungen in einer Menge von 50 bis 89 Gew.-%,

B) eine Gesamtmenge an anorganischen Füllstoffen in einer Menge von 10 bis 49 Gew.-% und

C) eine Gesamtmenge an Photoinitiatoren in einer Menge von 0,1 bis 5 Gew.-%,

jeweils bezogen auf die Gesamtmasse der polymerisierbaren Zusammensetzung,

dadurch gekennzeichnet, dass die Gesamtmenge (B) an anorganischen Füllstoffen

- eine erste Teilmenge (B1) an Dentalglas und

- eine zweite Teilmenge (B2) an pyrogener Kieselsäure

umfasst und wobei das Massenverhältnis von B1 zu B2 im Bereich von 10 : 1 bis 1 : 1 liegt.

In einer bevorzugten Ausführungsform liegt das Massenverhältnis von B1 zu B2 im Bereich von 5 : 1 bis 1,5 : 1, bevorzugt im Bereich von 3,5 : 1 bis 2 : 1.

In einer bevorzugten Ausführungsform umfasst die radikalisch polymerisierbare Zusammensetzung zum 3D-Druck von dentalen Kronen, Brücken, Prothesenzähnen oder Vollprothesen

A) in einer Menge von 56 bis 85 Gew.-%, bevorzugt 67 bis 84 Gew.-%,

B) in einer Menge von 10 bis 40 Gew.-%, bevorzugt 15 bis 30 Gew.-% und

C) in einer Menge von 0,1 bis 4 Gew.-%, bevorzugt 0,5 bis 3 Gew.-%,
jeweils bezogen auf die Gesamtmasse der polymerisierbaren Zusammensetzung.

Bevorzugt wird eine radikalisch polymerisierbare Zusammensetzung zum 3D-Druck von dentalen Kronen, Brücken, Prothesenzähnen oder Vollprothesen, wobei das Dentalglas B1

- eine mittlere Partikelgröße im Bereich von 0,4 bis 5 $\mu$m, bevorzugt 0,4 bis 2 $\mu$m, besonders bevorzugt im Bereich von 0,4 bis 1,0 $\mu$m, aufweist
und/oder (vorzugsweise "und")

- ausgewählt ist aus der Gruppe bestehend aus Bariumsilikatgläsern, Bariumaluminiumsilikatgläsern, Bariumborosilikatgläsern, Bariumboroaluminiumsilikatgläsern, Bariumborofluoroaluminiumsilikatgläsern, Strontiumsilikatgläsern, Strontiumaluminiumsilikatgläsern, Strontiumborosilikatgläsern, Strontiumboroaluminiumsilikatgläsern, Strontiumborofluoroaluminiumsilikatgläsern, und Zirkonsilikatgläsern, bevorzugt ausgewählt ist aus der Gruppe bestehend aus Bariumaluminiumsilikatgläsern, Bariumborosilikatgläsern und Bariumboroaluminiumsilikatgläsern, und/oder (vorzugsweise "und")

- organisch oberflächenmodifiziert, bevorzugt mit 3-Methacryloxy-propyl-(trimethoxy)silan organisch oberflächenmodifiziert, ist.

Bevorzugt wird eine radikalisch polymerisierbare Zusammensetzung zum 3D-Druck von dentalen Kronen, Brücken, Prothesenzähnen oder Vollprothesen, wobei
die Gesamtmenge (A) an polymerisierbaren (Meth)acryl-Verbindungen

- eine erste Teilmenge (A1) an difunktionellen, aromatischen (Meth)acrylaten,

- eine zweite Teilmenge (A2) an difunktionellen (Meth)acrylaten, die eine oder mehrere Urethan-Gruppen aufweisen, und

- eine dritte Teilmenge (A3) an monofunktionellen (Meth)acrylaten
umfasst.

In einer besonders bevorzugten Ausführungsform umfasst die radikalisch polymerisierbare Zusammensetzung zum 3D-Druck von dentalen Kronen, Brücken, Prothesenzähnen oder Vollprothesen

A1) in einer Menge von 20 bis 60 Gew.-%, bevorzugt 30 bis 50 Gew.-%,

A2) in einer Menge von 10 bis 40 Gew.-%, bevorzugt 15 bis 30 Gew.-% und

A3) in einer Menge von 1 bis 10 Gew.-%, bevorzugt 2 bis 8 Gew.-%,
und/oder (vorzugsweise "und")

B1) in einer Menge von 5 bis 44,5 Gew.-%, bevorzugt 6,7 bis 33,3 Gew.-%, besonders bevorzugt 10,7 bis 23,3 Gew.-%, und

B2) in einer Menge von 0,9 bis 24,5 Gew.-%, bevorzugt 1,7 bis 13,3 Gew.-%, besonders bevorzugt 3,3 bis 8,6 Gew.-%, jeweils bezogen auf die Gesamtmasse der polymerisierbaren Zusammensetzung.

In einer bevorzugten Ausführungsform ist

A1 ausgewählt aus der Gruppe bestehend aus 2,2-Bis[4-(meth)acryloyl-oxyethoxyphenyl]propan, 2,2-Bis[4-(meth)acryloyloxydiethoxy-phenyl]propan, 2,2-Bis[4-(meth)acryloyloxytriethoxyphenyl]propan, 2,2-Bis[4-(meth)acryloyloxytetraethoxyphenyl]-propan, 2,2-Bis[4-(meth)acryloyloxypenta-ethoxyphenyl]propan, 2,2-Bis[4-(meth)acryloyl-oxydipropoxyphenyl]propan, 2,2-Bis[4-(meth)acryloyloxyethoxyphenyl]-2-[4-(meth)-acryloyloxydiethoxyphenyl]propan, 2-[4-(Meth)acryloyloxydiethoxyphenyl]-2-[4-(meth)-acryloyloxytriethoxyphenyl] propan, 2-[4-(Meth)acryloyloxydipropoxyphenyl]-2-[4-(meth)-acryloyloxytriethoxyphenyl]propan, 2,2-Bis[4-(meth)acryloyloxyisopropoxyphenyl]propan, ethoxyliertes Bisphenol-A-di(meth)acrylat und propoxyliertes Bisphenol-A-di(meth)acrylat,
und/oder (vorzugsweise "und")

A2 ausgewählt aus der Gruppe bestehend aus 3,14-Dioxa-4,13-dioxo-5,12-diazahexadecan-1,16-dioxydi(meth)acrylat, 7,7,9-Trimethyl-3,14-dioxa-4,13-dioxo-5,12-diazahexadecan-1,16-dioxydi(meth)acrylat, 7,9,9-Trimethyl-3,14-dioxa-4,13-dioxo-5,12-diazahexadecan-1,16-dioxydi(meth)acrylat, und 1,5,5-Trimethyl-1-[(2-(meth)acryloyl-oxyethyl)carbamoylmethyl]-3-(2-(meth)acryloyloxyethyl)carbamoylcyclohexan (CAS 42405-01-6 Methacrylat; CAS 42404-50-2 Acrylat),
und/oder (vorzugsweise "und")

A3 ausgewählt aus der Gruppe bestehend aus 2-Hydroxyethyl(meth)acrylat, 2-Hydroxypropyl(meth)acrylat, 3-Hydroxypropyl(meth)acrylat, Tetrahydrofurfuryl(meth)acrylat, 4-(Meth)acryloylmorpholin, Cyclohexyl(meth)acrylat, 3,3,5-Trimethylcyclohexyl(meth)-acrylat, 4-(1,1-Dimethylethyl)cyclohexyl(meth)acrylat, Isobornyl(meth)acrylat, (Octahydro-4,7-methano-1H-indenyl)methyl(meth)acrylat, Benzyl(meth)acrylat, 2-Phenoxyethyl-(meth)acrylat, 2-(2-Phenoxyethoxy)ethyl(meth)acrylat, 2-[2-(2-Phenoxyethoxy)ethoxy]-ethyl(meth)acrylat, ethoxyliertes 2-Phenoxyethyl(meth)acrylat 2-Ethoxyethyl(meth)acrylat, 2-(2-Ethoxyethoxy)ethyl(meth)acrylat, 2-[2-(2-Ethoxyethoxy)ethoxy]ethyl(meth)acrylat, 2-Methoxyethyl(meth)acrylat, 2-(2-Methoxyethoxy)ethyl(meth)acrylat, 2-[2-(2-Methoxy-ethoxy)ethyl](meth)acrylat, Hexyl(meth)acrylat, 2-Ethylhexyl(meth)acrylat, Decyl-(meth)acrylat, Isodecyl(meth)acrylat, Dodecyl(meth)acrylat, 2-Hydroxy-3-(prop-2-enoyloxy)propyl 2-methyl-2-propylhexanoat und (5-Ethyl-1,3-dioxan-5-yl)methyl-(meth)-acrylat.

Radikalisch polymerisierbare (Meth)acryl-Verbindungen (A)

Eine erfindungsgemäße Zusammensetzung umfasst 50 bis 89 Gew.-%, bevorzugt 56 bis 85 Gew.-%, besonders bevorzugt 67 bis 84 Gew.-%, einer Gesamtmenge (A) an radikalisch polymerisierbaren (Meth)acryl-Verbindungen, jeweils bezogen auf die Gesamtmasse der polymerisierbaren Zusammensetzung.

Unter (Meth)acryl-Verbindungen werden im Rahmen der vorliegenden Anmeldung dabei sowohl Acryl-Verbindungen als auch Methacryl-Verbindungen verstanden.

In einer bevorzugten Ausführungsform umfasst die Gesamtmenge (A) an polymerisierbaren (Meth)acryl-Verbindungen

- eine erste Teilmenge (A1) an difunktionellen, aromatischen (Meth)acrylaten,

- eine zweite Teilmenge (A2) an difunktionellen (Meth)acrylaten, die eine oder mehrere Urethan-Gruppen aufweisen, und

- eine dritte Teilmenge (A3) an monofunktionellen (Meth)acrylaten.

In einer bevorzugten Ausführungsform umfasst die Gesamtmenge (A) an polymerisierbaren (Meth)acryl-Verbindungen

A1) in einer Menge von 20 bis 60 Gew.-%, bevorzugt 30 bis 50 Gew.-%,

A2) in einer Menge von 10 bis 40 Gew.-%, bevorzugt 15 bis 30 Gew.-% und

A3) in einer Menge von 1 bis 10 Gew.-%, bevorzugt 2 bis 8 Gew.-%,
jeweils bezogen auf die Gesamtmasse der polymerisierbaren Zusammensetzung.

Darüber hinaus kann eine erfindungsgemäße Zusammensetzung in der Gesamtmenge (A) auch eine vierte Teilmenge (A4) an polymerisierbaren (Meth)acryl-Verbindungen enthalten, die weder A1, A2 noch A3 entspricht.

Vorzugsweise umfasst die Gesamtmenge (A) an polymerisierbaren (Meth)acryl-Verbindungen einer solchen Zusammensetzung

A1) in einer Menge von 20 bis 60 Gew.-%, bevorzugt 30 bis 50 Gew.-%,

A2) in einer Menge von 10 bis 40 Gew.-%, bevorzugt 15 bis 30 Gew.-%,

A3) in einer Menge von 1 bis 10 Gew.-%, bevorzugt 2 bis 8 Gew.-% und

A4) in einer Menge von 0 bis 20 Gew.-%, bevorzugt 1 bis 8 Gew.-%,
jeweils bezogen auf die Gesamtmasse der polymerisierbaren Zusammensetzung.

Vorzugsweise umfasst die Teilmenge A1 eine oder mehrere (Meth)acryl-Verbindungen ausgewählt aus der Gruppe bestehend aus 2,2-Bis[4-(meth)acryloyloxyethoxy-phenyl]propan, 2,2-Bis[4-(meth)acryloyloxydiethoxyphenyl]propan, 2,2-Bis[4-(meth)-acryloyloxytriethoxyphenyl]propan, 2,2-Bis[4-(meth)acryloyloxytetraethoxyphenyl]propan, 2,2-Bis[4-(meth)acryloyloxypentaethoxyphenyl]propan, 2,2-Bis[4-(meth)acryloyloxydi-propoxyphenyl]propan, 2,2-Bis[4-(meth)acryloyloxyethoxyphenyl]-2-[4-(meth)acryloyloxy-diethoxyphenyl]propan, 2-[4-(Meth)acryloyloxydiethoxyphenyl]-2-[4-(meth)-acryloyloxytri-ethoxyphenyl]propan, 2-[4-(Meth)acryloyloxydipropoxyphenyl]-2-[4-(meth)acryloyloxy-triethoxyphenyl]propan, 2,2-Bis[4-(meth)acryloyloxyisopropoxyphenyl]propan, ethoxyliertes Bisphenol-A-di(meth)acrylat und propoxyliertes Bisphenol-A-di(meth)acrylat.

Vorzugsweise umfasst die Teilmenge A1 2,2-Bis[4-(2-hydroxy-3-(meth)acryloyloxy-propoxy)phenyl]propan (Bis-GMA) in einer Menge von weniger als 8 Gew.-%, bevorzugt weniger als 4 Gew.-%, besonders bevorzugt weniger als 2 Gew.-%, jeweils bezogen auf die Gesamtmasse der polymerisierbaren Zusammensetzung.

In einer bevorzugten Ausführungsform umfasst eine erfindungsgemäße Zusammensetzung im Wesentlichen kein 2,2-Bis[4-(2-hydroxy-3-(meth)acryloyloxy-propoxy)phenyl]propan oder gar kein 2,2-Bis[4-(2-hydroxy-3-(meth)acryloyloxy-propoxy)phenyl]propan.

Vorzugsweise umfasst die Teilmenge A2 eine oder mehrere (Meth)acryl-Verbindungen ausgewählt aus der Gruppe bestehend aus 3,14-Dioxa-4,13-dioxo-5,12-diazahexadecan-1,16-dioxydi(meth)acrylat, 7,7,9-Trimethyl-3,14-dioxa-4,13-dioxo-5,12-diazahexadecan-1,16-dioxydi(meth)acrylat, 7,9,9-Trimethyl-3,14-dioxa-4,13-dioxo-5,12-diazahexadecan-1,16-dioxydi(meth)acrylat, und 1,5,5-Trimethyl-1-[(2-(meth)acryloyloxyethyl)carbamoylmethyl]-3-(2-(meth)acryloyloxyethyl)carbamoylcyclohexan (CAS 42405-01-6 Methacrylat; CAS 42404-50-2 Acrylat).

Vorzugsweise umfasst die Teilmenge A3 eine oder mehrere (Meth)acryl-Verbindungen ausgewählt aus der Gruppe bestehend aus 2-Hydroxyethyl(meth)acrylat, 2-Hydroxypropyl(meth)acrylat, 3-Hydroxypropyl(meth)acrylat, Tetrahydrofurfuryl(meth)-acrylat, 4-(Meth)acryloylmorpholin, Cyclohexyl(meth)acrylat, 3,3,5-Trimethylcyclohexyl-(meth)acrylat, 4-(1,1-Dimethylethyl)cyclohexyl(meth)acrylat, Isobornyl(meth)acrylat, (Octahydro-4,7-methano-1H-indenyl)methyl(meth)acrylat, Benzyl(meth)acrylat, 2-Phenoxyethyl(meth)acrylat, 2-(2-Phenoxyethoxy)ethyl(meth)acrylat, 2-[2-(2-Phenoxy-ethoxy)ethoxy]ethyl(meth)acrylat, ethoxyliertes 2-Phenoxyethyl(meth)acrylat 2-Ethoxyethyl(meth)acrylat, 2-(2-Ethoxyethoxy)ethyl(meth)acrylat, 2-[2-(2-Ethoxyethoxy)ethoxy]-ethyl(meth)acrylat, 2-Methoxyethyl(meth)acrylat, 2-(2-Methoxyethoxy)ethyl(meth)acrylat, 2-[2-(2-Methoxyethoxy)ethoxy]ethyl(meth)acrylat, Hexyl(meth)acrylat, 2-Ethylhexyl-(meth)acrylat, Decyl(meth)acrylat, Isodecyl(meth)acrylat, Dodecyl(meth)acrylat, 2-Hydroxy-3-(prop-2-enoyloxy)propyl 2-methyl-2-propylhexanoat und (5-Ethyl-1,3-dioxan-5-yl)methyl-(meth)acrylat.

Vorzugsweise umfasst die Teilmenge A4 eine oder mehrere (Meth)acryl-Verbindungen ausgewählt aus der Gruppe bestehend aus Ethylenglycoldi(meth)acrylat, Diethylen-glycoldi(meth)acrylat, Triethylenglycoldi(meth)acrylat, Tetraethylenglycoldi(meth)acrylat, Polyethylenglycoldi(meth)acrylat, Propylenglycoldi(meth)acrylat, Dipropylenglycol-di(meth)acrylat, Tripropylenglycoldi(meth)acrylat, Tetrapropylenglycoldi(meth)acrylat, Polypropylenglycol-di(meth)acrylat, 1,4-Butandioldi(meth)acrylat, 1,3-Butandioldi(meth)-acrylat, 1,6-Hexandioldi(meth)acrylat, 1,10-Decandioldi(meth)acrylat, 1,12-Dodecandioldi-(meth)acrylat, 2-Hydroxypropyl-1,3-di(meth)acrylat, 3-Hydroxypropyl-1,2-di(meth)acrylat, Neopentylglycoldi(meth)acrylat und 3(4),8(9)-Bis((meth)acryloyloxymethyl)tricyclo-[5.2.1.0$^{2,6}$]decan.

Anorganische Füllstoffe (B)

Eine erfindungsgemäße Zusammensetzung umfasst 10 bis 49 Gew.-%, bevorzugt 10 bis 40 Gew.-%, besonders bevorzugt 15 bis 30 Gew.-%, einer Gesamtmenge (B) an anorganischen Füllstoffen, jeweils bezogen auf die Gesamtmasse der polymerisierbaren Zusammensetzung.

Die Gesamtmenge (B) an anorganischen Füllstoffen umfasst dabei eine erste Teilmenge (B1) an Dentalglas und eine zweite Teilmenge (B2) an pyrogener Kieselsäure, wobei das Massenverhältnis von B1 zu B2 im Bereich von 10 : 1 bis 1 : 1, bevorzugt im Bereich von 5 : 1 bis 1,5 : 1, besonders bevorzugt im Bereich von 3,5 : 1 bis 2 : 1, liegt.

Unter Dentalglas werden Gläser verstanden, die für den Einsatz im Dentalbereich geeignet sind. Sie zeichnen sich u.a. durch eine hohe Reinheit aus und enthalten keine giftigen Bestandteile (z.B. Blei oder Arsen). Solche Dentalgläser sind u.a. von SCHOTT unter den Bezeichnungen G018-307, G018-163, G018-093, GM39923, GM32087, G018-308, GM27884, GM31685, G018-053, 8235, G018-186, GM31684, G018-310, G018-159, G018-161, GM35429, G018-090 und G018-117 oder von FERRO unter den Bezeichnungen IS 50 1101, IS 50 1102 und IS 50 1103 kommerziell in unterschiedlichen Korngrößen erhältlich.

Vorzugsweise enthält eine erfindungsgemäße Zusammensetzung B1 in einer Menge von 5 bis 44,5 Gew.-%, bevorzugt 6,7 bis 33,3 Gew.-%, besonders bevorzugt 10,7 bis 23,3 Gew.-%, jeweils bezogen auf die Gesamtmasse der polymerisierbaren Zusammensetzung.

Vorzugsweise ist B1 ausgewählt ist aus der Gruppe bestehend aus Bariumsilikatgläsern, Bariumaluminiumsilikatgläsern, Bariumborosilikatgläsern, Bariumboroaluminiumsilikatgläsern, Bariumborofluoroaluminiumsilikatgläsern, Strontiumsilikatgläsern, Strontiumaluminiumsilikatgläsern, Strontiumborosilikatgläsern, Strontiumboroaluminiumsilikatgläsern, Strontiumborofluoroaluminiumsilikatgläsern, und Zirkonsilikatgläsern, bevorzugt ausgewählt ist aus der Gruppe bestehend aus Bariumaluminiumsilikatgläsern, Bariumborosilikatgläsern und Bariumboroaluminiumsilikatgläsern.

In einer bevorzugten Ausführungsform weist B1 eine mittlere Partikelgröße im Bereich von 0,4 bis 5 μm, bevorzugt 0,4 bis 2 μm, besonders bevorzugt im Bereich von 0,4 bis 1,0 μm, auf.

Die mittlere Partikelgröße bezieht sich dabei auf mittels statischer Lichtstreuung bestimmte d50-Werte bei volumengewichteter Auswertung.

In einer weiteren bevorzugten Ausführungsform ist B1 organisch oberflächenmodifiziert, bevorzugt mit 3-Methacryloxypropyl(trimethoxy)silan organisch oberflächenmodifiziert.

Vorzugsweise enthält eine erfindungsgemäße Zusammensetzung B2 in einer Menge von 0,9 bis 24,5 Gew.-%, bevorzugt 1,7 bis 13,3 Gew.-%, besonders bevorzugt 3,3 bis 8,6 Gew.-%, jeweils bezogen auf die Gesamtmasse der polymerisierbaren Zusammensetzung.

In einer bevorzugten Ausführungsform weist die pyrogene Kieselsäure B2 eine Primärpartikelgröße im Bereich von 5 bis 25 nm, vorzugsweise im Bereich von 10 bis 20 nm, auf.

Die Primärpartikelgrößen von B2 lassen sich mittels Transmissionselektronenmikroskopie (TEM) bestimmen. Entsprechende Verfahren sind in "Untersuchungen zur fließregulierenden Eigenschaft hochdisperser Fällungskieselsäuren" (Anne-Kathrin Müller, Dissertation, Würzburg 2008) beschrieben.

In einer weiteren bevorzugten Ausführungsform ist die pyrogene Kieselsäure B2 organisch oberflächenmodifiziert, vorzugsweise mit 3-Methacryloxypropyl(trimethoxy)silan, Hexamethyldisilazan oder Dichlor(dimethyl)silan organisch oberflächenmodifiziert.

In einer weiteren bevorzugten Ausführungsform weist die pyrogene Kieselsäure B2 eine BET-Oberfläche im Bereich von 100 bis 300 $m^2$/g, vorzugsweise im Bereich von 120 bis 200 $m^2$/g, auf. Vorzugsweise wird die BET-Oberfläche entsprechend DIN ISO 9277:2014 bestimmt.

In einer weiteren bevorzugten Ausführungsform weist die pyrogene Kieselsäure B2 eine Stampfdichte im Bereich von 100 bis 300 g/L, vorzugsweise im Bereich von 120 bis 250 g/L, auf. Vorzugsweise wird die Stampfdichte entsprechend DIN ISO 787-11:1995 bestimmt.

In einer weiteren bevorzugten Ausführungsform ist die pyrogene Kieselsäure B2 strukturmodifiziert und/oder verdichtet und nachvermahlen, vorzugsweise strukturmodifiziert durch Verdichtung und Nachvermahlung.

Unter Strukturmodifizierung wird ein Verfahren zur Strukturänderung (i.d.R. zum Strukturabbau) von pyrogenen Metalloxiden, insbesondere pyrogenen Kieselsäuren, verstanden, bei dem das mikroskopische 3D-Netzwerk der Aggregate und Agglomerate derart verändert, dass Materialien mit neuen Eigenschaften entstehen. So zeichnen sich diese vor allem durch eine reduzierte Verdickungswirkung und erhöhte Stampfdichte aus. Ein geeignetes Verfahren zur Strukturmodifizierung von pyrogenen Kieselsäuren wird in DE 196 16 781 A1 (Degussa AG) beschrieben.

Photoinitiatoren (C)

Eine erfindungsgemäße Zusammensetzung enthält 0,1 bis 5 Gew.-%, bevorzugt 0,1 bis 4 Gew.-%, besonders bevorzugt 0,5 bis 3 Gew.-%, jeweils bezogen auf die Gesamtmasse der polymerisierbaren Zusammensetzung, einer Gesamtmenge an Photoinitiatoren (C).

Als Photoinitiatoren können die für (Meth)acryl-Systeme üblichen, geeigneten Verbindungen eingesetzt werden. Vorteilhaft werden alpha-Diketone, Benzoinalkylether, Thioxanthone, Benzophenone, Acetophenone, Acylphosphinoxide oder Acylgermanium-Verbindungen eingesetzt. Bevorzugt werden Monoacylphosphinoxide oder Bisacylphosphinoxide verwendet.

In einer bevorzugten Ausgestaltung ist (C) ausgewählt aus der Gruppe bestehend aus alpha-Diketonen, Benzoinalkylethern, Thioxanthonen, Benzophenonen, Acetophenonen, Acylphosphinoxiden, und Acylgermanium-Verbindungen, bevorzugt ausgewählt aus der Gruppe bestehend aus 1-Hydroxycyclohexylbenzophenon, 4-(2-Hydroxyethoxy)phenyl-(2-hydroxy-2-propyl)keton, 2-Hydroxy-2-methylpropiophenon, 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate (CAS 84434-11-7) und (Poly(oxy-1,2-ethandiyl),$\alpha,\alpha',\alpha$"-1,2,3-propantriyltris[$\omega$-[[phenyl(2,4,6-trimethylbenzoyl)phosphinyl]oxy]-Polymer) (CAS 1834525-17-5), besonders bevorzugt ausgewählt aus der Gruppe bestehend aus 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate und (Poly(oxy-1,2-ethandiyl),$\alpha,\alpha',\alpha$"-1,2,3-propantriyltris[$\omega$-[[phenyl(2,4,6-trimethylbenzoyl)phosphinyl]oxy]-Polymer).

In einer bevorzugten Ausführungsform umfasst eine erfindungsgemäße Zusammensetzung zusätzlich 0,001 bis 1 Gew.-%, bevorzugt 0,01 bis 0,5 Gew.-%, besonders bevorzugt 0,01 bis 0,3 Gew.-%, jeweils bezogen auf die Gesamtmasse der polymerisierbaren Zusammensetzung, eines oder mehrerer Stabilisatoren (D).

Vorzugsweise ist (D) ausgewählt aus der Gruppe bestehend aus Hydrochinon, Hydrochinonmonomethylether, 2,6-Di-*tert*.-butyl-4-methylphenol, *tert*.-Butylhydroxyanisol und 2,2,6,6-Tetramethyl-piperidin-1-oxyl.

Um die Farbe des Materials für die gewünschte Anwendung einzustellen kann eine erfindungsgemäße Zusammensetzung zusätzlich ein oder mehrere Farbmittel (E) enthalten. Als Farbmittel können anorganische Farbpigmente sowie organische Farbpigmente oder Farbstoffe eingesetzt werden. Für zahnfarbene Anwendungen werden weiße, gelbe, orange, rote, braune und/oder schwarze Farbmittel eingesetzt.

Vorzugsweise umfasst (E) Titandioxid und/oder Eisenoxid.

In einer bevorzugten Ausführungsform enthält eine erfindungsgemäße Zusammensetzung 0,0001 bis 1 Gew.-%, bevorzugt 0,0001 bis 0,5 Gew.-%, besonders bevorzugt 0,0001 bis 0,1 Gew.-%, jeweils bezogen auf die Gesamtmasse der polymerisierbaren Zusammensetzung, eines oder mehrerer Farbmittel (E).

In einer besonders bevorzugten Ausführungsform liegt die Viskosität der radikalisch polymerisierbare Zusammensetzung zum 3D-Druck von dentalen Kronen, Brücken, Prothesenzähnen oder Vollprothesen

- bei einer Scherrate von 0,01 s$^{-1}$ im Bereich von 100 bis 500 Pa*s, bevorzugt im Bereich von 200 bis 270 Pa*s, und/oder (vorzugsweise "und")

- bei einer Scherrate von 0,1 s$^{-1}$ im Bereich von 10 bis 50 Pa*s, bevorzugt im Bereich von 30 bis 40 Pa*s, und/oder (vorzugsweise "und")

- bei einer Scherrate von 1 s$^{-1}$ im Bereich von 2 bis 20 Pa*s, bevorzugt im Bereich von 6,5 bis 10,5 Pa*s, und/oder (vorzugsweise "und")

- bei einer Scherrate von 10 s$^{-1}$ im Bereich von 1 bis 10 Pa*s, bevorzugt im Bereich von 2,5 bis 6,0 Pa*s.

Der Verlustfaktor tan $\delta$ ist der Quotient aus Verlustmodul G'' (Imaginärteil) und Speichermodul G' (Realteil). Er beschreibt in der Rheologie das viskoelastische Verhalten. Je niedriger der Verlustfaktor, desto mehr entspricht das Verhalten einer Probe dem eines ideal-elastischen Festkörpers. Je höher der Verlustfaktor, desto mehr nähert sich das Verhalten einer Probe dem einer ideal-viskosen Flüssigkeit mit newtonschem Fließverhalten an. Bei tan $\delta$ = 1 findet ein Übergang von einem Gel zu einer Flüssigkeit statt. Es ist daher bevorzugt, wenn erfindungsgemäße Zusammensetzungen bei geringer Scherbelastung (kleine Auslenkungen bis 0,01%), wie sie im Ruhezustand z.B. während der Lagerung in der Flasche oder Harzwanne vorkommt, einen tan $\delta$ von kleiner 1 aufweisen. Sie zeigen dann einen Gelcharakter, der in einer guten Sedimentationsstabilität resultiert. Zudem ist es bevorzugt, wenn erfindungsgemäße Zusammensetzungen bei höherer Scherbelastung (größere Auslenkungen über 0,1%), wie sie insbesondere während des 3D-Druck-Vorgangs im Bereich der bewegten Bauplattform vorkommt, einen tan $\delta$ von größer 1 aufweisen. Sie verhalten sich dann wie Flüssigkeiten, können gut nachfließen und sorgen für eine hohe Präzision beim 3D-Druck.

Bevorzugt wird daher eine radikalisch polymerisierbare Zusammensetzung zum 3D-Druck von dentalen Kronen, Brücken, Prothesenzähnen oder Vollprothesen, wobei der Quotient tan $\delta$ aus Verlustmodul G'' und Speichermodul G' bei oszillierender Scherbelastung

- bei Auslenkungen im Bereich von 0,001 bis 0,01% kleiner als 1 ist, bevorzugt im Bereich von 0,4 bis 0,99 liegt, besonders bevorzugt im Bereich von 0,6 bis 0,95 liegt, und
- bei Auslenkungen im Bereich von 1% bis 10% größer als 1 ist, bevorzugt im Bereich von 1,01 bis 5 liegt, besonders bevorzugt im Bereich von 1,05 bis 3 liegt.

Erfindungsgemäß ist auch ein(e) dentale(r) Krone, Brücke, Prothesenzahn oder Vollprothese, der/die durch 3D-Druck einer erfindungsgemäßen radikalisch polymerisierbaren Zusammensetzung hergestellt wurde.

Erfindungsgemäß ist auch eine radikalisch polymerisierbare Zusammensetzung zur Anwendung in einem therapeutischen Verfahren, vorzugsweise in einem therapeutischen Verfahren als

- temporäre Krone oder Brücke,

- permanente Krone oder Brücke,

- Prothesenzahn,

- Prothesenbasis,

- Teilprothese oder

- Vollprothese.

Beispiele:

Die Ziele und Vorteile dieser Offenbarung werden durch die folgenden Beispiele weiter veranschaulicht, aber die spezifischen Materialien und deren Mengen, die in diesen Beispielen genannt werden, sowie andere Bedingungen und Details sollten nicht so ausgelegt werden, dass sie diese Offenbarung unangemessen einschränken.

Die in den Beispielen verwendeten Materialien werden im Folgenden zusammengefasst.

Bis-EMA-4:    ethoxyliertes Bisphenol A Dimethacrylat (4 EO-Einheiten; CAS 41637-38-1)

(fortgesetzt)

| | |
|---|---|
| TMDI-UDMA | 7,7,9-Trimethyl-3,14-dioxa-4,13-dioxo-5,12-diazahexadecan-1,16-dioxydi-(meth)acrylat / 7,9,9-Trimethyl-3,14-dioxa-4,13-dioxo-5,12-diazahexa-decan-1,16-dioxydi(meth)acrylat (Isomerengemisch; CAS 72869-86-4) |
| IPDI-UDMA | 1,5,5-Trimethyl-1-[(2-methacryloyloxyethyl)carbamoylmethyl]-3-(2-methacryloyloxyethyl)carbamoylcyclohexan (CAS 42405-01-6) |
| TEDMA: | Triethylenglycoldimethacrylat (CAS 109-16-0) |
| DDDMA: | 1,10-Decandioldimethacrylat (CAS 6701-13-9) |
| IBOMA: | Isobornylmethacrylat (CAS 7534-94-3) |
| PheMA | 2-Phenoxyethylmethacrylat (CAS 10595-06-9) |
| DG1: | Dentalglas G018-308 (0,7 $\mu$m; Oberflächenmodifizierung 3-MPS) |
| DG2: | Dentalglas IS 50 1101 (0,4 $\mu$m; Oberflächenmodifizierung 3-MPS) |
| DG3: | Dentalglas IS 50 1101 (1,0 $\mu$m; Oberflächenmodifizierung 3-MPS) |
| DG4: | Dentalglas G018-308 (2,0 $\mu$m; Oberflächenmodifizierung 3-MPS) |
| PK1: | pyrogene Kieselsäure (Primärpartikel 12 nm; BET 145 m$^2$/g; Stampfdichte 188 g/L; Oberflächenmodifizierung HMDS; hergestellt entsprechend dem Beispiel aus DE 196 16 781 A1) |
| PK2: | pyrogene Kieselsäure (Primärpartikel 12 nm; BET 160 m$^2$/g; Stampfdichte 140 g/L; Oberflächenmodifizierung HMDS) |
| PK3: | pyrogene Kieselsäure (Primärpartikel 12 nm; BET 150 m$^2$/g; Stampfdichte 230 g/L; Oberflächenmodifizierung 3-MPS) |
| PK4: | Aerosil A200 (Primärpartikel 12 nm; BET 200 m$^2$/g; Stampfdichte 50 g/L) |
| PK5: | Aerosil R816 (Primärpartikel 12 nm; BET 190 m$^2$/g; Stampfdichte 60 g/L; Oberflächenmodifizierung HMDS) |
| HMDS: | Hexamethyldisilazan (CAS 999-97-3) |
| 3-MPS: | 3-Methacryloxypropyl(trimethoxy)silan (CAS 2530-85-0) |
| TPO: | Diphenyl(2,4,6-trimethylbenzoyl)phosphinoxid (CAS 75980-60-8) |
| BHT: | 2,6-Di-*tert*.-butyl-4-methylphenol (CAS 128-37-0) |

Messmethoden:

Biegefestigkeit (BF): Die Biegefestigkeiten wurden angepasst an ISO 4049:2009 bestimmt. Aus den Druckharzen wurden mittels 3D-Druck (SolFlex350, W2P Engineering GmbH; Wellenlänge 385 nm, Leistung 8.3 mW/cm$^2$, Pixelgröße 50 $\mu$m, Schichtstärke 50 $\mu$m) Prüfkörper mit der Abmessung 25 mm x 2 mm x 2 mm hergestellt und mit einem Otoflash (VOCO GmbH) mit 2x2000 Blitzen nachbelichtet. Die Bestimmung der Biegefestigkeit erfolgt bei einer Vorschubgeschwindigkeit von 1 mm/min an einer Zwick-Universalprüfmaschine (Zwick GmbH & Co. KG, Ulm).

Elastizitäsmodul (E-Modul): Der Elastizitätsmodul wurde analog der Berechnung in ADA Spec. No. 27:1993 (7.8.4.2) als Steigung der Spannung-Dehnungs-Kurve der 3-Punkt-Biegefestigkeitsmessung im linear-elastischen Bereich ermittelt.

$$E = \frac{3\,L}{4\,b\,h^3}\frac{\Delta F}{\Delta d}$$

(L: Stützweite; b: Probenbreite; h: Probendicke; $\Delta d$: Deformation im linear-elastischen Bereich; $\Delta F$: Kraftänderung bei einer Deformation $\Delta d$)

Viskosität: Die Viskosität wird an einem Rheometer Physica MCR 301 (Anton Paar, Graz) mit einer 50 mm-Messplatte (Platte/Platte), 0,5 mm Spaltabstand und 1 g Substanz gemessen. Vor der Messung wird die Platte auf eine Temperatur von 23 °C temperiert. Die Messdauer beträgt 30 s bei einer Scherrate von 10/s.

tan $\delta$: Der Verlustfaktor tan $\delta$ (G''/G') wird an einem Rheometer Physica MCR 301 (Anton Paar, Graz) mit einer 50 mm-Messplatte (Kegel/Platte; Winkel 1°), 0,10 mm Spaltabstand und 0,2 g Substanz bei 23 °C bestimmt. Die Deformation (Auslenkung) wird logarithmisch von 0,0001% bis 10% mit 10 rad/s erhöht. Es werden 6 Messpunkte pro Dekade aufgenommen. Ausgewertet werden die Messpunkte bei 0,01% und 1 % Deformation.

Zentrifugenstabilität: Zur Beurteilung der Zentrifugenstabilität wurden in einer Labor-Standard-"EBA 20" Zentrifuge von Rettich 3 g der jeweiligen Zusammensetzungen in ein Zentrifugenglas, das einen Durchmesser von 15 mm sowie eine Länge von 100 mm hatte, eingewogen. Anschließend wurden die Zusammensetzungen bei 6000 U/min dreimal 5 Minuten zentrifugiert. Die Temperatur während der Zentrifugenläufe betrug 23°C.

Partikelgröße: Die Partikelgrößenverteilung der Füllstoffe B1 wurde mittels statischer Lichtstreuung an einem Beckman Coulter LS 13 320 bestimmt. Die angegebenen Partikelgrößen sind die d50-Werte bei volumengewichteter Auswertung.

Herstellung der 3D-Druck-Zusammensetzungen: Für die Herstellung der entsprechenden Zusammensetzungen wurden zunächst die Monomere, Initiatoren und Inhibitoren in den nachfolgend angegebenen Verhältnissen eingewogen und jeweils für zwei Stunden bei Raumtemperatur gerührt, bis die Feststoffe vollständig gelöst waren und homogene Lösungen entstanden waren. Anschließend wurden die Füllstoffe (Dentalglas, pyrogene Kieselsäure, Farbpigmente) in den angegebenen Verhältnissen zugegeben. Anschließend wurden die Mischungen an einem SpeedMixer DAC 800 (Hauschild GmbH & Co. KG, Hamm) viermal für jeweils 30 Sekunden bei 1500 U/min homogenisiert. Anschließend wurden die Mischungen am SpeedMixer DAC 800 für weitere 30 Sekunden bei 1000 U/min und -0,9 bar entlüftet.

Tabelle 1:

| Beispiel | | 1 | 2 | 3 | 4 |
|---|---|---|---|---|---|
| A1 | Bis-EMA-4 | 45.0 | 45.0 | 45.0 | 45.0 |
| A2 | TMDI-UDMA | 20.0 | | 20.0 | |
| | IPDI-UDMA | | 20.0 | | 20.0 |
| A3 | IBOMA | 7.3 | 7.3 | | |
| | PheMA | | | 7.3 | 7.3 |
| B1 | DG1 | 20.0 | 20.0 | 20.0 | 20.0 |
| B2 | PK1 | 6.0 | 6.0 | 6.0 | 6.0 |
| C | TPO | 1.5 | 1.5 | 1.5 | 1.5 |
| D | BHT | 0.1 | 0.1 | 0.1 | 0.1 |
| E | $TiO_2$ | 0.1 | 0.1 | 0.1 | 0.1 |
| Biegefestigkeit [MPa] | | 107 | 109 | 103 | 105 |
| E-Modul [GPa] | | 3.3 | 3.3 | 3.1 | 3.1 |
| Viskosität (10/s) [mPa*s] | | 4500 | 4600 | 4650 | 4700 |
| tan $\delta$ (0.01 %) | | 0.82 | 0.82 | 0.84 | 0.85 |
| tan $\delta$ (1%) | | 1.51 | 1.52 | 1.46 | 1.45 |
| Zentrifugenstabilität | | i.O. | i.O. | i.O. | i.O. |

Tabelle 2:

| Beispiel | | 5 | 6 | 7 | 8 |
|---|---|---|---|---|---|
| A1 | Bis-EMA-4 | 42.5 | 40.0 | 42.5 | 40.0 |
| A2 | IPDI-UDMA | 20.0 | 20.0 | 20.0 | 20.0 |
| A3 | IBOMA | 6.8 | 6.3 | 6.8 | 6.3 |
| A4 | TEDMA | 3.0 | 6.0 | | |
| | DDDMA | | | 3.0 | 6.0 |
| B1 | DG1 | 20.0 | 20.0 | 20.0 | 20.0 |
| B2 | PK1 | 6.0 | 6.0 | 6.0 | 6.0 |
| C | TPO | 1.5 | 1.5 | 1.5 | 1.5 |
| D | BHT | 0.1 | 0.1 | 0.1 | 0.1 |

(fortgesetzt)

| Beispiel | | 5 | 6 | 7 | 8 |
|---|---|---|---|---|---|
| E | TiO$_2$ | 0.1 | 0.1 | 0.1 | 0.1 |
| Biegefestigkeit [MPa] | | 110 | 113 | 108 | 109 |
| E-Modul [GPa] | | 3.7 | 3.7 | 3.4 | 3.4 |
| Viskosität (10/s) [mPa*s] | | 4820 | 4940 | 4800 | 4910 |
| tan $\delta$ (0.01 %) | | 0.74 | 0.70 | 0.79 | 0.76 |
| tan $\delta$ (1%) | | 1.67 | 1.66 | 1.59 | 1.59 |
| Zentrifugenstabilität | | i.O. | i.O. | i.O. | i.O. |

Tabelle 3:

| Beispiel | | 9 | 10 | 11 | 12 |
|---|---|---|---|---|---|
| A1 | Bis-EMA-4 | 49.5 | 45.0 | 40.0 | 35.0 |
| A2 | IPDI-UDMA | 27.0 | 25.0 | 18.0 | 12.0 |
| A3 | IBOMA | 7.1 | 7.3 | 7.3 | 4.5 |
| A4 | TEDMA | | | 3.4 | 4.0 |
| B1 | DG1 | 10.7 | 15.0 | 23.0 | 33.3 |
| B2 | PK1 | 4.0 | 6.0 | 6.6 | 9.5 |
| C | TPO | 1.5 | 1.5 | 1.5 | 1.5 |
| D | BHT | 0.1 | 0.1 | 0.1 | 0.1 |
| E | TiO$_2$ | 0.1 | 0.1 | 0.1 | 0.1 |
| Biegefestigkeit [MPa] | | 100 | 101 | 115 | 123 |
| E-Modul [GPa] | | 2.5 | 2.9 | 4.2 | 4.8 |
| Viskosität (10/s) [mPa*s] | | 2520 | 3130 | 5550 | 6000 |
| tan $\delta$ (0.01 %) | | 0.91 | 0.89 | 0.63 | 0.60 |
| tan $\delta$ (1%) | | 1.90 | 1.83 | 1.23 | 1.05 |
| Zentrifugenstabilität | | i.O. | i.O. | i.O. | i.O. |

Tabelle 4:

| Beispiel | | 13 | 14 | 15 | 16 |
|---|---|---|---|---|---|
| A1 | Bis-EMA-4 | 45.0 | 45.0 | 45.0 | 45.0 |
| A2 | IPDI-UDMA | 20.0 | 20.0 | 20.0 | 20.0 |
| A3 | IBOMA | 7.3 | 7.3 | 7.3 | 7.3 |
| B1 | DG1 | 20.0 | 20.0 | 20.0 | 20.0 |
| B2 | PK2 | 6.0 | | | |
| | PK3 | | 6.0 | | |
| | PK4 | | | 6.0 | |
| | PK5 | | | | 6.0 |
| C | TPO | 1.5 | 1.5 | 1.5 | 1.5 |

(fortgesetzt)

| Beispiel | | 13 | 14 | 15 | 16 |
|---|---|---|---|---|---|
| D | BHT | 0.1 | 0.1 | 0.1 | 0.1 |
| E | TiO$_2$ | 0.1 | 0.1 | 0.1 | 0.1 |
| Biegefestigkeit [MPa] | | 110 | 108 | 98 | 99 |
| E-Modul [GPa] | | 3.3 | 3.3 | 3.1 | 3.1 |
| Viskosität (10/s) [mPa*s] | | 4620 | 4650 | 4640 | 4650 |
| tan δ (0.01 %) | | 0.83 | 0.84 | 1.05 | 1.08 |
| tan δ (1%) | | 1.52 | 1.51 | 1.52 | 1.50 |
| Zentrifugenstabilität | | i.O. | i.O. | * | * |
| * nach zwei Durchläufen noch i.O.; nach dem dritten Durchlauf leichte Sedimentation erkennbar | | | | | |

Tabelle 5:

| Beispiel | | 17 | 18 | 19 | 20 | 21 |
|---|---|---|---|---|---|---|
| A1 | Bis-EMA-4 | 45.0 | 45.0 | 45.0 | 45.0 | 45.0 |
| A2 | IPDI-UDMA | 20.0 | 22.0 | 19.0 | 19.0 | 20.0 |
| A3 | IBOMA | 7.3 | 7.3 | 7.3 | 7.3 | 7.3 |
| B1 | DG2 | 20.0 | 18.0 | | | |
| | DG3 | | | 21.0 | 21.0 | |
| | DG4 | | | | | 20.0 |
| B2 | PK1 | 6.0 | 6.0 | 6.0 | 6.0 | 6.0 |
| C | TPO | 1.5 | 1.5 | 1.5 | 1.5 | 1.5 |
| D | BHT | 0.1 | 0.1 | 0.1 | 0.1 | 0.1 |
| E | TiO$_2$ | 0.1 | 0.1 | 0.1 | 0.1 | 0.1 |
| Biegefestigkeit [MPa] | | 118 | 111 | 102 | 106 | 1101 |
| E-Modul [GPa] | | 3.9 | 3.5 | 2.9 | 3.1 | 3.1 |
| Viskosität (10/s) [mPa*s] | | 5100 | 4920 | 2970 | 3110 | 3750 |
| tan δ (0.01%) | | 0.67 | 0.72 | 0.92 | 0.89 | 0.95 |
| tan δ (1%) | | 1.22 | 1.30 | 1.88 | 1.69 | 1.03 |
| Zentrifugenstabilität | | i.O. | i.O. | i.O. | i.O. | * |
| * nach zwei Durchläufen noch i.O.; nach dem dritten Durchlauf leichte Sedimentation erkennbar | | | | | | |

Tabelle 6:

| Beispiel | | 22 (Vergleich) | 23 (Vergleich) | 24 (Vergleich) | 25 (Vergleich) |
|---|---|---|---|---|---|
| A1 | Bis-EMA-4 | 45.0 | 49.8 | 55.0 | 45.0 |
| A2 | IPDI-UDMA | 25.0 | 20.0 | 30.0 | 20.0 |
| A3 | IBOMA | 6.5 | 6.5 | 4.3 | 6.8 |
| B1 | DG1 | 20.0 | 10.0 | 7.0 | 26.5 |
| B2 | PK1 | 1.8 | 12.0 | 2.0 | |

14

(fortgesetzt)

| Beispiel | | 22 (Vergleich) | 23 (Vergleich) | 24 (Vergleich) | 25 (Vergleich) |
|---|---|---|---|---|---|
| C | TPO | 1.5 | 1.5 | 1.5 | 1.5 |
| D | BHT | 0.1 | 0.1 | 0.1 | 0.1 |
| E | TiO$_2$ | 0.1 | 0.1 | 0.1 | 0.1 |
| Biegefestigkeit [MPa] | | 97 | 88 | 83 | 99 |
| E-Modul [GPa] | | 2.1 | 1.8 | 1.4 | 2.3 |
| Viskosität (10/s) [mPa*s] | | 2080 | 1920 | 1450 | 1820 |
| tan δ (0.01%) | | 1.05 | 1.24 | 1.33 | 1.26 |
| tan δ (1%) | | 1.89 | 2.20 | 2.95 | 2.30 |
| Zentrifugenstabilität | | nein ** | nein ** | nein ** | nein *** |
| ** Material bereits nach einem Durchlauf sedimentiert. *** Material bereits nach einem Durchlauf sedimentiert. Zudem ist bereits bei einer Lagerung von zwei Wochen bei 23 °C eine Sedimentierung erkennbar. | | | | | |

Insgesamt lässt sich die Erfindung anhand der folgenden Aspekte zusammenfassen.

1. Radikalisch polymerisierbare Zusammensetzung zum 3D-Druck von dentalen Kronen, Brücken, Prothesenzähnen oder Vollprothesen umfassend

A) eine Gesamtmenge an radikalisch polymerisierbaren (Meth)acryl-Verbindungen in einer Menge von 50 bis 89 Gew.-%,
B) eine Gesamtmenge an anorganischen Füllstoffen in einer Menge von 10 bis 49 Gew.-% und
C) eine Gesamtmenge an Photoinitiatoren in einer Menge von 0,1 bis 5 Gew.-%,

jeweils bezogen auf die Gesamtmasse der polymerisierbaren Zusammensetzung,

dadurch gekennzeichnet, dass die Gesamtmenge (B) an anorganischen Füllstoffen

- eine erste Teilmenge (B1) an Dentalglas
  und

- eine zweite Teilmenge (B2) an pyrogener Kieselsäure

umfasst und wobei das Massenverhältnis von B1 zu B2 im Bereich von 10 : 1 bis 1 : 1 liegt.

2. Radikalisch polymerisierbare Zusammensetzung zum 3D-Druck von dentalen Kronen, Brücken, Prothesenzähnen oder Vollprothesen nach Aspekt 1, wobei das Massenverhältnis von B1 zu B2 im Bereich von 5 : 1 bis 1,5 : 1, bevorzugt im Bereich von 3,5 : 1 bis 2 : 1, liegt.

3. Radikalisch polymerisierbare Zusammensetzung zum 3D-Druck von dentalen Kronen, Brücken, Prothesenzähnen oder Vollprothesen nach einem der vorangehenden Aspekte, umfassend

A) in einer Menge von 56 bis 85 Gew.-%, bevorzugt 67 bis 84 Gew.-%,
B) in einer Menge von 10 bis 40 Gew.-%, bevorzugt 15 bis 30 Gew.-% und
C) in einer Menge von 0,1 bis 4 Gew.-%, bevorzugt 0,5 bis 3 Gew.-%,
jeweils bezogen auf die Gesamtmasse der polymerisierbaren Zusammensetzung.

4. Radikalisch polymerisierbare Zusammensetzung zum 3D-Druck von dentalen Kronen, Brücken, Prothesenzähnen oder Vollprothesen nach einem der vorangehenden Aspekte, wobei das Dentalglas B1 eine mittlere Partikelgröße im Bereich von 0,4 bis 5 μm, bevorzugt 0,4 bis 2 μm, besonders bevorzugt im Bereich von 0,4 bis 1,0 μm, aufweist.

5. Radikalisch polymerisierbare Zusammensetzung zum 3D-Druck von dentalen Kronen, Brücken, Prothesenzähnen oder Vollprothesen nach einem der vorangehenden Aspekte, wobei die mittlere Partikelgröße von B1 mittels statischer Lichtstreuung als d50-Wert bei volumengewichteter Auswertung bestimmt wird.

6. Radikalisch polymerisierbare Zusammensetzung zum 3D-Druck von dentalen Kronen, Brücken, Prothesenzähnen oder Vollprothesen nach einem der vorangehenden Aspekte, wobei das Dentalglas B1 ausgewählt ist aus der Gruppe bestehend aus Bariumsilikatgläsern, Bariumaluminiumsilikatgläsern, Bariumborosilikatgläsern, Bariumboroaluminiumsilikatgläsern, Bariumborofluoroaluminiumsilikatgläsern, Strontiumsilikatgläsern, Strontiumaluminiumsilikatgläsern, Strontiumborosilikatgläsern, Strontiumboroaluminiumsilikatgläsern, Strontiumborofluoroaluminiumsilikatgläsern, und Zirkonsilikatgläsern, bevorzugt ausgewählt ist aus der Gruppe bestehend aus Bariumaluminiumsilikatgläsern, Bariumborosilikatgläsern und Bariumboroaluminiumsilikatgläsern.

7. Radikalisch polymerisierbare Zusammensetzung zum 3D-Druck von dentalen Kronen, Brücken, Prothesenzähnen oder Vollprothesen nach einem der vorangehenden Aspekte, wobei das Dentalglas B1 organisch oberflächenmodifiziert, bevorzugt mit 3-Methacryloxypropyl(trimethoxy)silan organisch oberflächenmodifiziert, ist.

8. Radikalisch polymerisierbare Zusammensetzung zum 3D-Druck von dentalen Kronen, Brücken, Prothesenzähnen oder Vollprothesen nach einem der vorangehenden Aspekte, wobei die pyrogene Kieselsäure B2 eine Primärpartikelgröße im Bereich von 5 bis 25 nm, vorzugsweise im Bereich von 10 bis 20 nm, aufweist.

9. Radikalisch polymerisierbare Zusammensetzung zum 3D-Druck von dentalen Kronen, Brücken, Prothesenzähnen oder Vollprothesen nach einem der vorangehenden Aspekte, wobei die pyrogene Kieselsäure B2 eine Primärpartikelgröße im Bereich von 5 bis 25 nm, vorzugsweise im Bereich von 10 bis 20 nm, aufweist, wobei die Primärpartikelgröße als volumengewichtete Verteilung mittels Transmissionselektronenmikroskopie bestimmt wird.

10. Radikalisch polymerisierbare Zusammensetzung zum 3D-Druck von dentalen Kronen, Brücken, Prothesenzähnen oder Vollprothesen nach einem der vorangehenden Aspekte, wobei die pyrogene Kieselsäure B2 organisch oberflächenmodifiziert, vorzugsweise mit 3-Methacryloxypropyl(trimethoxy)silan, Hexamethyldisilazan oder Dichlor(dimethyl)silan organisch oberflächenmodifiziert, ist.

11. Radikalisch polymerisierbare Zusammensetzung zum 3D-Druck von dentalen Kronen, Brücken, Prothesenzähnen oder Vollprothesen nach einem der vorangehenden Aspekte, wobei die pyrogene Kieselsäure B2 eine BET-Oberfläche im Bereich von 100 bis 300 $m^2$/g, vorzugsweise im Bereich von 120 bis 200 $m^2$/g, aufweist.

12. Radikalisch polymerisierbare Zusammensetzung zum 3D-Druck von dentalen Kronen, Brücken, Prothesenzähnen oder Vollprothesen nach einem der vorangehenden Aspekte, wobei die pyrogene Kieselsäure B2 eine BET-Oberfläche im Bereich von 100 bis 300 $m^2$/g, vorzugsweise im Bereich von 120 bis 200 $m^2$/g, aufweist, wobei die BET-Oberfläche nach DIN ISO 9277:2014 bestimmt wird.

13. Radikalisch polymerisierbare Zusammensetzung zum 3D-Druck von dentalen Kronen, Brücken, Prothesenzähnen oder Vollprothesen nach einem der vorangehenden Aspekte, wobei die pyrogene Kieselsäure B2 eine Stampfdichte im Bereich von 100 bis 300 g/L, vorzugsweise im Bereich von 120 bis 250 g/L, aufweist.

14. Radikalisch polymerisierbare Zusammensetzung zum 3D-Druck von dentalen Kronen, Brücken, Prothesenzähnen oder Vollprothesen nach einem der vorangehenden Aspekte, wobei die pyrogene Kieselsäure B2 eine Stampfdichte im Bereich von 100 bis 300 g/L, vorzugsweise im Bereich von 120 bis 250 g/L, aufweist, wobei die Stampfdichte nach DIN ISO 787-11:1995 bestimmt wird.

15. Radikalisch polymerisierbare Zusammensetzung zum 3D-Druck von dentalen Kronen, Brücken, Prothesenzähnen oder Vollprothesen nach einem der vorangehenden Aspekte, wobei die pyrogene Kieselsäure B2 strukturmodifiziert und/oder verdichtet und nachvermahlen, vorzugsweise strukturmodifiziert durch Verdichtung und Nachvermahlung, ist.

16. Radikalisch polymerisierbare Zusammensetzung zum 3D-Druck von dentalen Kronen, Brücken, Prothesenzähnen oder Vollprothesen nach einem der vorangehenden Aspekte, wobei
die Gesamtmenge (A) an polymerisierbaren (Meth)acryl-Verbindungen

- eine erste Teilmenge (A1) an difunktionellen, aromatischen (Meth)acrylaten,

- eine zweite Teilmenge (A2) an difunktionellen (Meth)acrylaten, die eine oder mehrere Urethan-Gruppen aufweisen, und
- eine dritte Teilmenge (A3) an monofunktionellen (Meth)acrylaten umfasst.

17. Radikalisch polymerisierbare Zusammensetzung zum 3D-Druck von dentalen Kronen, Brücken, Prothesenzähnen oder Vollprothesen nach einem der vorangehenden Aspekte, umfassend

A1) in einer Menge von 20 bis 60 Gew.-%, bevorzugt 30 bis 50 Gew.-%,
A2) in einer Menge von 10 bis 40 Gew.-%, bevorzugt 15 bis 30 Gew.-% und
A3) in einer Menge von 1 bis 10 Gew.-%, bevorzugt 2 bis 8 Gew.-%,
jeweils bezogen auf die Gesamtmasse der polymerisierbaren Zusammensetzung.

18. Radikalisch polymerisierbare Zusammensetzung zum 3D-Druck von dentalen Kronen, Brücken, Prothesenzähnen oder Vollprothesen nach einem der vorangehenden Aspekte, wobei die Gesamtmenge (A) an polymerisierbaren (Meth)acryl-Verbindungen eine vierte Teilmenge (A4) an polymerisierbaren (Meth)acryl-Verbindungen, die weder A1, A2 noch A3 entsprechen, umfasst.

19. Radikalisch polymerisierbare Zusammensetzung zum 3D-Druck von dentalen Kronen, Brücken, Prothesenzähnen oder Vollprothesen nach einem der vorangehenden Aspekte, umfassend

A1) in einer Menge von 20 bis 60 Gew.-%, bevorzugt 30 bis 50 Gew.-%,
A2) in einer Menge von 10 bis 40 Gew.-%, bevorzugt 15 bis 30 Gew.-%,
A3) in einer Menge von 1 bis 10 Gew.-%, bevorzugt 2 bis 8 Gew.-% und
A4) in einer Menge von 0 bis 20 Gew.-%, bevorzugt 1 bis 8 Gew.-%,
jeweils bezogen auf die Gesamtmasse der polymerisierbaren Zusammensetzung.

20. Radikalisch polymerisierbare Zusammensetzung zum 3D-Druck von dentalen Kronen, Brücken, Prothesenzähnen oder Vollprothesen nach einem der vorangehenden Aspekte, umfassend

B1) in einer Menge von 5 bis 44,5 Gew.-%, bevorzugt 6,7 bis 33,3 Gew.-%, besonders bevorzugt 10,7 bis 23,3 Gew.-%, und
B2) in einer Menge von 0,9 bis 24,5 Gew.-%, bevorzugt 1,7 bis 13,3 Gew.-%, besonders bevorzugt 3,3 bis 8,6 Gew.-%,
jeweils bezogen auf die Gesamtmasse der polymerisierbaren Zusammensetzung.

21. Radikalisch polymerisierbare Zusammensetzung zum 3D-Druck von dentalen Kronen, Brücken, Prothesenzähnen oder Vollprothesen nach einem der vorangehenden Aspekte, wobei A1 ausgewählt ist aus der Gruppe bestehend aus 2,2-Bis[4-(meth)-acryloyloxyethoxyphenyl]propan, 2,2-Bis[4-(meth)acryloyloxydiethoxy-phenyl]propan, 2,2-Bis[4-(meth)acryloyloxytriethoxyphenyl]propan, 2,2-Bis[4-(meth)acryloyloxytetra-ethoxyphenyl]propan, 2,2-Bis[4-(meth)acryloyloxypenta-ethoxyphenyl]-propan, 2,2-Bis[4-(meth)acryloyloxydipropoxyphenyl]propan, 2,2-Bis[4-(meth)acryloyloxyethoxy-phenyl]-2-[4-(meth)acryloyloxydiethoxyphenyl]propan, 2-[4-(Meth)acryloyloxy-diethoxyphenyl]-2-[4-(meth)-acryloyloxytriethoxyphenyl]propan, 2-[4-(Meth)acryloyl-oxydipropoxyphenyl]-2-[4-(meth)acryloyloxytriethoxyphenyl]propan, 2,2-Bis[4-(meth)-acryloyloxyisopropoxyphenyl]propan, ethoxyliertes Bisphenol-A-di(meth)acrylat und propoxyliertes Bisphenol-A-di(meth)acrylat.

22. Radikalisch polymerisierbare Zusammensetzung zum 3D-Druck von dentalen Kronen, Brücken, Prothesenzähnen oder Vollprothesen nach einem der vorangehenden Aspekte, wobei die Teilmenge A1 2,2-Bis[4-(2-hydroxy-3-(meth)acryloyloxy-propoxy)phenyl]propan (Bis-GMA) in einer Menge von weniger als 8 Gew.-%, bevorzugt weniger als 4 Gew.-%, besonders bevorzugt weniger als 2 Gew.-%, jeweils bezogen auf die Gesamtmasse der polymerisierbaren Zusammensetzung, umfasst.

23. Radikalisch polymerisierbare Zusammensetzung zum 3D-Druck von dentalen Kronen, Brücken, Prothesenzähnen oder Vollprothesen nach einem der vorangehenden Aspekte, wobei die Teilmenge A1 im Wesentlichen kein 2,2-Bis[4-(2-hydroxy-3-(meth)acryloyloxypropoxy)phenyl]propan oder gar kein 2,2-Bis[4-(2-hydroxy-3-(meth)acryloyloxypropoxy)phenyl]propan umfasst.

24. Radikalisch polymerisierbare Zusammensetzung zum 3D-Druck von dentalen Kronen, Brücken, Prothesenzähnen oder Vollprothesen nach einem der vorangehenden Aspekte, wobei A2 ausgewählt ist aus der Gruppe bestehend aus 3,14-Dioxa-4,13-dioxo-5,12-diazahexadecan-1,16-dioxydi(meth)acrylat, 7,7,9-Trimethyl-3,14-dioxa-4,13-dioxo-5,12-diazahexadecan-1,16-dioxydi(meth)acrylat, 7,9,9-Trimethyl-3,14-di-oxa-4,13-dioxo-5,12-diazahexadecan-1,16-dioxydi(meth)acrylat und 1,5,5-Trimethyl-1-[(2-(meth)acryloyloxyethyl)carbamoylmethyl]-3-(2-(meth)acryloyloxyethyl)carba-moylcyclohexan (CAS 42405-01-6 Methacrylat; CAS 42404-50-2 Acrylat).

25. Radikalisch polymerisierbare Zusammensetzung zum 3D-Druck von dentalen Kronen, Brücken, Prothesenzähnnen oder Vollprothesen nach einem der vorangehenden Aspekte, wobei A3 ausgewählt ist aus der Gruppe bestehend aus 2-Hydroxyethyl(meth)acrylat, 2-Hydroxypropyl(meth)acrylat, 3-Hydroxypropyl(meth)acrylat, Tetrahydrofurfuryl(meth)acrylat, 4-(Meth)acryloylmorpholin, Cyclohexyl(meth)acrylat, 3,3,5-Trimethylcyclohexyl(meth)acrylat, 4-(1,1-Dimethylethyl)cyclohexyl(meth)-acrylat, Isobornyl(meth)acrylat, (Octahydro-4,7-methano-1H-indenyl)methyl(meth)-acrylat, Benzyl(meth)acrylat, 2-Phenoxyethyl(meth)acrylat, 2-(2-Phenoxyethoxy)-ethyl(meth)acrylat, 2-[2-(2-Phenoxyethoxy)ethoxy]ethyl(meth)acrylat, ethoxyliertes 2-Phenoxyethyl(meth)acrylat 2-Ethoxyethyl(meth)acrylat, 2-(2-Ethoxyethoxy)ethyl-(meth)acrylat, 2-[2-(2-Ethoxyethoxy)ethoxy]ethyl(meth)acrylat, 2-Methoxyethyl-(meth)acrylat, 2-(2-Methoxyethoxy)ethyl(meth)acrylat, 2-[2-(2-Methoxyethoxy)-ethoxy]ethyl(meth)acrylat, Hexyl(meth)acrylat, 2-Ethylhexyl(meth)acrylat, Decyl-(meth)acrylat, Isodecyl(meth)acrylat, Dodecyl(meth)acrylat, 2-Hydroxy-3-(prop-2-enoyloxy)propyl 2-methyl-2-propylhexanoat und (5-Ethyl-1,3-dioxan-5-yl)methyl-(meth)acrylat.

26. Radikalisch polymerisierbare Zusammensetzung zum 3D-Druck von dentalen Kronen, Brücken, Prothesenzähnnen oder Vollprothesen nach einem der vorangehenden Aspekte, wobei A3 eine monofunktionelle (Meth)acryl-Verbindung mit einer alicyclischen Rest umfasst.

27. Radikalisch polymerisierbare Zusammensetzung zum 3D-Druck von dentalen Kronen, Brücken, Prothesenzähnnen oder Vollprothesen nach einem der vorangehenden Aspekte, wobei A4 ausgewählt ist aus der Gruppe bestehend aus Ethylenglycoldi(meth)acrylat, Diethylenglycoldi(meth)acrylat, Triethylenglycol-di(meth)acrylat, Tetraethylenglycoldi(meth)acrylat, Polyethylenglycoldi(meth)acrylat, Propylenglycoldi(meth)acrylat, Dipropylenglycoldi(meth)acrylat, Tripropylen-glycoldi(meth)acrylat, Tetrapropylenglycoldi(meth)acrylat, Polypropylenglycoldi(meth)acrylat, 1,4-Butandioldi(meth)acrylat, 1,3-Butandioldi(meth)acrylat, 1,6-Hexandioldi(meth)acrylat, 1,10-Decandioldi(meth)acrylat, 1,12-Dodecandioldi-(meth)acrylat, 2-Hydroxypropyl-1,3-di(meth)acrylat, 3-Hydroxypropyl-1,2-di(meth)-acrylat, Neopentylglycoldi(meth)acrylat und 3(4),8(9)-Bis((meth)acryloyloxymethyl)-tricyclo[5.2.1.0$^{2,6}$]decan.

28. Radikalisch polymerisierbare Zusammensetzung zum 3D-Druck von dentalen Kronen, Brücken, Prothesenzähnnen oder Vollprothesen nach einem der vorangehenden Aspekte, umfassend eine Gesamtmenge an Photoinitiatoren (C) in einer Menge von 0,1 bis 5 Gew.-%, bevorzugt 0,1 bis 4 Gew.-%, besonders bevorzugt 0,5 bis 3 Gew.-%, jeweils bezogen auf die Gesamtmasse der polymerisierbaren Zusammensetzung.

29. Radikalisch polymerisierbare Zusammensetzung zum 3D-Druck von dentalen Kronen, Brücken, Prothesenzähnnen oder Vollprothesen nach einem der vorangehenden Aspekte, wobei (C) ausgewählt ist aus der Gruppe bestehend aus alpha-Diketonen, Benzoinalkylethern, Thioxanthonen, Benzophenonen, Acetophenonen, Acylphosphinoxiden, und Acylgermanium-Verbindungen, bevorzugt ausgewählt ist aus der Gruppe bestehend aus 1-Hydroxycyclohexylbenzophenon, 4-(2-Hydroxyethoxy)phenyl-(2-hydroxy-2-propyl)keton, 2-Hydroxy-2-methylpropiophenon, 2,4,6-Trimethylbenzoyldiphenylphosphinoxid, Phenyl-bis(2,4,6-trimethylbenzoyl)-phosphinoxid, Ethyl phenyl(2,4,6-trimethylbenzoyl)phosphinate (CAS 84434-11-7) und (Poly(oxy-1,2-ethandiyl),α,α',α''-1,2,3-propantriyltris[ω-[[phenyl(2,4,6-trimethylbenzoyl)phosphinyl]oxy]-Polymer) (CAS 1834525-17-5), besonders bevorzugt ausgewählt ist aus der Gruppe bestehend aus 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate und (Poly(oxy-1,2-ethandiyl),α,α',α''-1,2,3-propantriyltris[ω-[[phenyl(2,4,6-trimethylbenzoyl)phosphinyl]oxy]-Polymer).

30. Radikalisch polymerisierbare Zusammensetzung zum 3D-Druck von dentalen Kronen, Brücken, Prothesenzähnnen oder Vollprothesen nach einem der vorangehenden Aspekte, umfassend eine Gesamtmenge an Stabilisatoren (D) in einer Menge von 0,001 bis 1 Gew.-%, bevorzugt 0,01 bis 0,5 Gew.-%, besonders bevorzugt 0,01 bis 0,3 Gew.-%, jeweils bezogen auf die Gesamtmasse der polymerisierbaren Zusammensetzung.

31. Radikalisch polymerisierbare Zusammensetzung zum 3D-Druck von dentalen Kronen, Brücken, Prothesenzähnnen oder Vollprothesen nach einem der vorangehenden Aspekte, wobei (D) ausgewählt ist aus der Gruppe bestehend aus Hydrochinon, Hydrochinonmonomethylether, 2,6-Di-*tert*.-butyl-4-methylphenol, *tert*.-Butylhydroxyanisol und 2,2,6,6-Tetramethyl-piperidin-1-oxyl.

32. Radikalisch polymerisierbare Zusammensetzung zum 3D-Druck von dentalen Kronen, Brücken, Prothesenzähnen oder Vollprothesen nach einem der vorangehenden Aspekte, umfassend eine Gesamtmenge an Farbmitteln (E) in einer Menge von 0,0001 bis 1 Gew.-%, bevorzugt 0,0001 bis 0,5 Gew.-%, besonders bevorzugt 0,0001 bis 0,1 Gew.-%, jeweils bezogen auf die Gesamtmasse der polymerisierbaren Zusammensetzung.

33. Radikalisch polymerisierbare Zusammensetzung zum 3D-Druck von dentalen Kronen, Brücken, Prothesenzähnen oder Vollprothesen nach einem der vorangehenden Aspekte, wobei (E) Titandioxid und/oder Eisenoxid umfasst.

34. Radikalisch polymerisierbare Zusammensetzung zum 3D-Druck von dentalen Kronen, Brücken, Prothesenzähnen oder Vollprothesen nach einem der vorangehenden Aspekte, wobei die Viskosität bei einer Scherrate von 0,01 $s^{-1}$ im Bereich von 100 bis 500 Pa*s, bevorzugt im Bereich von 200 bis 270 Pa*s, liegt.

35. Radikalisch polymerisierbare Zusammensetzung zum 3D-Druck von dentalen Kronen, Brücken, Prothesenzähnen oder Vollprothesen nach einem der vorangehenden Aspekte, wobei die Viskosität bei einer Scherrate von 0,1 $s^{-1}$ im Bereich von 10 bis 50 Pa*s, bevorzugt im Bereich von 30 bis 40 Pa*s, liegt.

36. Radikalisch polymerisierbare Zusammensetzung zum 3D-Druck von dentalen Kronen, Brücken, Prothesenzähnen oder Vollprothesen nach einem der vorangehenden Aspekte, wobei die Viskosität bei einer Scherrate von 1 $s^{-1}$ im Bereich von 2 bis 20 Pa*s, bevorzugt im Bereich von 6,5 bis 10,5 Pa*s, liegt.

37. Radikalisch polymerisierbare Zusammensetzung zum 3D-Druck von dentalen Kronen, Brücken, Prothesenzähnen oder Vollprothesen nach einem der vorangehenden Aspekte, wobei die Viskosität bei einer Scherrate von 10 $s^{-1}$ im Bereich von 1 bis 10 Pa*s, bevorzugt im Bereich von 2,5 bis 6,0 Pa*s, liegt.

38. Radikalisch polymerisierbare Zusammensetzung zum 3D-Druck von dentalen Kronen, Brücken, Prothesenzähnen oder Vollprothesen nach einem der vorangehenden Aspekte, wobei der Quotient tan δ aus Verlustmodul G" und Speichermodul G' bei oszillierender Scherbelastung

- bei Auslenkungen im Bereich von 0,001% bis 0,01% kleiner als 1 ist, bevorzugt im Bereich von 0,4 bis 0,99 liegt, besonders bevorzugt im Bereich von 0,6 bis 0,95 liegt, und
- bei Auslenkungen im Bereich von 1% bis 10% größer als 1 ist, bevorzugt im Bereich von 1,01 bis 5 liegt, besonders bevorzugt im Bereich von 1,05 bis 3 liegt.

39. Radikalisch polymerisierbare Zusammensetzung zum 3D-Druck von dentalen Kronen, Brücken, Prothesenzähnen oder Vollprothesen nach einem der vorangehenden Aspekte, wobei der Quotient tan δ aus Verlustmodul G" und Speichermodul G' bei oszillierender Scherbelastung

- bei Auslenkungen ≤0,01% kleiner als 1 ist, bevorzugt im Bereich von 0,4 bis 0,99 liegt, besonders bevorzugt im Bereich von 0,6 bis 0,95 liegt, und
- bei Auslenkungen ≥1% größer als 1 ist, bevorzugt im Bereich von 1,01 bis 5 liegt, besonders bevorzugt im Bereich von 1,05 bis 3 liegt.

40. Dentale Krone oder Brücke hergestellt durch 3D-Druck einer radikalisch polymerisierbaren Zusammensetzung nach einem der Aspekte 1 bis 39.

41. Dentaler Prothesenzahn hergestellt durch 3D-Druck einer radikalisch polymerisierbaren Zusammensetzung nach einem der Aspekte 1 bis 39.

42. Dentale Prothesenbasis, Teilprothese oder Vollprothese hergestellt durch 3D-Druck einer radikalisch polymerisierbaren Zusammensetzung nach einem der Aspekte 1 bis 39.

43. Radikalisch polymerisierbare Zusammensetzung nach einem der Aspekte 1 bis 39 zur Anwendung in einem therapeutischen Verfahren, vorzugsweise in einem therapeutischen Verfahren als

- temporäre Krone oder Brücke,
- permanente Krone oder Brücke,

- Prothesenzahn,
- Prothesenbasis,
- Teilprothese oder
- Vollprothese.

**Patentansprüche**

1. Radikalisch polymerisierbare Zusammensetzung zum 3D-Druck von dentalen Kronen, Brücken, Prothesenzähnen oder Vollprothesen umfassend

   A) eine Gesamtmenge an radikalisch polymerisierbaren (Meth)acryl-Verbindungen in einer Menge von 50 bis 89 Gew.-%,
   B) eine Gesamtmenge an anorganischen Füllstoffen in einer Menge von 10 bis 49 Gew.-% und
   C) eine Gesamtmenge an Photoinitiatoren in einer Menge von 0,1 bis 5 Gew.-%,

   jeweils bezogen auf die Gesamtmasse der polymerisierbaren Zusammensetzung,
   **dadurch gekennzeichnet, dass** die Gesamtmenge (B) an anorganischen Füllstoffen

   - eine erste Teilmenge (B1) an Dentalglas
   und
   - eine zweite Teilmenge (B2) an pyrogener Kieselsäure

   umfasst und wobei das Massenverhältnis von B1 zu B2 im Bereich von 10 : 1 bis 1 : 1 liegt.

2. Radikalisch polymerisierbare Zusammensetzung zum 3D-Druck von dentalen Kronen, Brücken, Prothesenzähnen oder Vollprothesen nach Anspruch 1, wobei das Massenverhältnis von B1 zu B2 im Bereich von 5 : 1 bis 1,5 : 1, bevorzugt im Bereich von 3,5 : 1 bis 2 : 1, liegt.

3. Radikalisch polymerisierbare Zusammensetzung zum 3D-Druck von dentalen Kronen, Brücken, Prothesenzähnen oder Vollprothesen nach einem der vorangehenden Ansprüche, umfassend

   A) in einer Menge von 56 bis 85 Gew.-%, bevorzugt 67 bis 84 Gew.-%,
   B) in einer Menge von 10 bis 40 Gew.-%, bevorzugt 15 bis 30 Gew.-% und
   C) in einer Menge von 0,1 bis 4 Gew.-%, bevorzugt 0,5 bis 3 Gew.-%,
   jeweils bezogen auf die Gesamtmasse der polymerisierbaren Zusammensetzung.

4. Radikalisch polymerisierbare Zusammensetzung zum 3D-Druck von dentalen Kronen, Brücken, Prothesenzähnen oder Vollprothesen nach einem der vorangehenden Ansprüche, wobei das Dentalglas B1

   - eine mittlere Partikelgröße im Bereich von 0,4 bis 5 $\mu$m, bevorzugt 0,4 bis 2 $\mu$m, besonders bevorzugt im Bereich von 0,4 bis 1,0 $\mu$m, aufweist
   und/oder
   - ausgewählt ist aus der Gruppe bestehend aus Bariumsilikatgläsern, Bariumaluminiumsilikatgläsern, Bariumborosilikatgläsern, Bariumboroaluminiumsilikatgläsern, Bariumborofluoroaluminiumsilikatgläsern, Strontiumsilikatgläsern, Strontiumaluminiumsilikatgläsern, Strontiumborosilikatgläsern, Strontiumboroaluminiumsilikatgläsern, Strontiumborofluoroaluminiumsilikatgläsern, und Zirkonsilikatgläsern, bevorzugt ausgewählt ist aus der Gruppe bestehend aus Bariumaluminiumsilikatgläsern, Bariumborosilikatgläsern und Bariumboroaluminiumsilikatgläsern,
   und/oder
   - organisch oberflächenmodifiziert, bevorzugt mit 3-Methacryloxy-propyl(trimethoxy)silan organisch oberflächenmodifiziert, ist.

5. Radikalisch polymerisierbare Zusammensetzung zum 3D-Druck von dentalen Kronen, Brücken, Prothesenzähnen oder Vollprothesen nach einem der vorangehenden Ansprüche, wobei die pyrogene Kieselsäure B2 eine Primärpartikelgröße im Bereich von 5 bis 25 nm, vorzugsweise im Bereich von 10 bis 20 nm, aufweist.

6. Radikalisch polymerisierbare Zusammensetzung zum 3D-Druck von dentalen Kronen, Brücken, Prothesenzähnen oder Vollprothesen nach einem der vorangehenden Ansprüche, wobei die pyrogene Kieselsäure B2 organisch oberflächenmodifiziert, vorzugsweise mit 3-Methacryloxypropyl(trimethoxy)silan, Hexamethyldisilazan oder Dichlor(dimethyl)silan organisch oberflächenmodifiziert, ist.

7. Radikalisch polymerisierbare Zusammensetzung zum 3D-Druck von dentalen Kronen, Brücken, Prothesenzähnen oder Vollprothesen nach einem der vorangehenden Ansprüche, wobei die pyrogene Kieselsäure B2 eine BET-Oberfläche im Bereich von 100 bis 300 m²/g, vorzugsweise im Bereich von 120 bis 200 m²/g, aufweist.

8. Radikalisch polymerisierbare Zusammensetzung zum 3D-Druck von dentalen Kronen, Brücken, Prothesenzähnen oder Vollprothesen nach einem der vorangehenden Ansprüche, wobei die pyrogene Kieselsäure B2 eine Stampfdichte im Bereich von 100 bis 300 g/L, vorzugsweise im Bereich von 120 bis 250 g/L, aufweist.

9. Radikalisch polymerisierbare Zusammensetzung zum 3D-Druck von dentalen Kronen, Brücken, Prothesenzähnen oder Vollprothesen nach einem der vorangehenden Ansprüche, wobei die pyrogene Kieselsäure B2 strukturmodifiziert und/oder verdichtet und nachvermahlen, vorzugsweise strukturmodifiziert durch Verdichtung und Nachvermahlung, ist.

10. Radikalisch polymerisierbare Zusammensetzung zum 3D-Druck von dentalen Kronen, Brücken, Prothesenzähnen oder Vollprothesen nach einem der vorangehenden Ansprüche, wobei die Gesamtmenge (A) an polymerisierbaren (Meth)acryl-Verbindungen

- eine erste Teilmenge (A1) an difunktionellen, aromatischen (Meth)acrylaten,
- eine zweite Teilmenge (A2) an difunktionellen (Meth)acrylaten, die eine oder mehrere Urethan-Gruppen aufweisen, und
- eine dritte Teilmenge (A3) an monofunktionellen (Meth)acrylaten
umfasst.

11. Radikalisch polymerisierbare Zusammensetzung zum 3D-Druck von dentalen Kronen, Brücken, Prothesenzähnen oder Vollprothesen nach einem der vorangehenden Ansprüche, umfassend

A1) in einer Menge von 20 bis 60 Gew.-%, bevorzugt 30 bis 50 Gew.-%,
A2) in einer Menge von 10 bis 40 Gew.-%, bevorzugt 15 bis 30 Gew.-% und
A3) in einer Menge von 1 bis 10 Gew.-%, bevorzugt 2 bis 8 Gew.-%,
und/oder
B1) in einer Menge von 5 bis 44,5 Gew.-%, bevorzugt 6,7 bis 33,3 Gew.-%, besonders bevorzugt 10,7 bis 23,3 Gew.-%, und
B2) in einer Menge von 0,9 bis 24,5 Gew.-%, bevorzugt 1,7 bis 13,3 Gew.-%, besonders bevorzugt 3,3 bis 8,6 Gew.-%,
jeweils bezogen auf die Gesamtmasse der polymerisierbaren Zusammensetzung.

12. Radikalisch polymerisierbare Zusammensetzung zum 3D-Druck von dentalen Kronen, Brücken, Prothesenzähnen oder Vollprothesen nach einem der vorangehenden Ansprüche, wobei

A1 ausgewählt ist aus der Gruppe bestehend aus 2,2-Bis[4-(meth)acryloyloxyethoxy-phenyl]propan, 2,2-Bis[4-(meth)acryloyloxydiethoxyphenyl]propan, 2,2-Bis[4-(meth)-acryloyloxytriethoxyphenyl]propan, 2,2-Bis[4-(meth)acryloyloxytetraethoxyphenyl]-propan, 2,2-Bis[4-(meth)acryloyloxypentaethoxyphenyl]propan, 2,2-Bis[4-(meth)-acryloyloxydipropoxyphenyl]propan, 2,2-Bis[4-(meth)acryloyloxyethoxyphenyl]-2-[4-(meth)acryloyloxydiethoxyphenyl]propan, 2-[4-(Meth)acryloyloxydiethoxyphenyl]-2-[4-(meth)acryloyloxy-triethoxyphenyl]propan, 2-[4-(Meth)acryloyloxydipropoxyphenyl]-2-[4-(meth)acryloyloxytriethoxyphenyl]propan, 2,2-Bis[4-(meth)acryloyloxyisopro-poxyphenyl]propan, ethoxyliertes Bisphenol-A-di(meth)acrylat und propoxyliertes Bisphenol-A-di(meth)acrylat,
und/oder
A2 ausgewählt ist aus der Gruppe bestehend aus 3,14-Dioxa-4,13-dioxo-5,12-diazahexadecan-1,16-dioxydi(meth)acrylat, 7,7,9-Trimethyl-3,14-dioxa-4,13-dioxo-5,12-diazahexadecan-1,16-dioxydi(meth)acrylat, 7,9,9-Trimethyl-3,14-dioxa-4,13-dioxo-5,12-diazahexadecan-1,16-dioxydi(meth)acrylat und 1,5,5-Trimethyl-1-[(2-(meth)acryloyloxyethyl)carbamoylmethyl]-3-(2-(meth)acryloyloxyethyl)-carbamoyl-cyclohexan (CAS 42405-01-6 Methacrylat; CAS 42404-50-2 Acrylat),
und/oder

A3 ausgewählt ist aus der Gruppe bestehend aus 2-Hydroxyethyl(meth)acrylat, 2-Hydroxypropyl(meth)acrylat, 3-Hydroxypropyl(meth)acrylat, Tetrahydrofurfuryl(meth)-acrylat, 4-(Meth)acryloylmorpholin, Cyclohexyl(meth)acrylat, 3,3,5-Trimethylcyclo-hexyl(meth)acrylat, 4-(1,1-Dimethylethyl)cyclohexyl(meth)acrylat, Isobornyl(meth)-acrylat, (Octahydro-4,7-methano-1H-indenyl)-methyl(meth)acrylat, Benzyl(meth)-acrylat, 2-Phenoxyethyl(meth)acrylat, 2-(2-Phenoxyethoxy)ethyl(meth)acrylat, 2-[2-(2-Phenoxyethoxy)ethoxy]ethyl(meth)acrylat, ethoxyliertes 2-Phenoxyethyl(meth)-acrylat 2-Ethoxyethyl(meth)acrylat, 2-(2-Ethoxyethoxy)ethyl(meth)acrylat, 2-[2-(2-Ethoxyethoxy)ethoxy]ethyl(meth)acrylat, 2-Methoxyethyl(meth)acrylat, 2-(2-Methoxy-ethoxy)ethyl(meth)acrylat, 2-[2-(2-Methoxyethoxy)ethoxy]ethyl(meth)acrylat, Hexyl-(meth)acrylat, 2-Ethylhexyl(meth)acrylat, Decyl(meth)acrylat, Isodecyl(meth)acrylat, Dodecyl(meth)acrylat, 2-Hydroxy-3-(prop-2-enoyloxy)propyl 2-methyl-2-propylhexa-noat und (5-Ethyl-1,3-dioxan-5-yl)methyl-(meth)acrylat.

13. Radikalisch polymerisierbare Zusammensetzung zum 3D-Druck von dentalen Kronen, Brücken, Prothesenzähnen oder Vollprothesen nach einem der vorangehenden Ansprüche, wobei die Viskosität

- bei einer Scherrate von 0,01 s$^{-1}$ im Bereich von 100 bis 500 Pa*s, bevorzugt im Bereich von 200 bis 270 Pa*s, und/oder
- bei einer Scherrate von 0,1 s$^{-1}$ im Bereich von 10 bis 50 Pa*s, bevorzugt im Bereich von 30 bis 40 Pa*s, und/oder
- bei einer Scherrate von 1 s$^{-1}$ im Bereich von 2 bis 20 Pa*s, bevorzugt im Bereich von 6,5 bis 10,5 Pa*s, und/oder
- bei einer Scherrate von 10 s$^{-1}$ im Bereich von 1 bis 10 Pa*s, bevorzugt im Bereich von 2,5 bis 6,0 Pa*s, liegt.

14. Radikalisch polymerisierbare Zusammensetzung zum 3D-Druck von dentalen Kronen, Brücken, Prothesenzähnen oder Vollprothesen nach einem der vorangehenden Ansprüche, wobei der Quotient tan δ aus Verlustmodul G" und Speichermodul G' bei oszillierender Scherbelastung

- bei Auslenkungen im Bereich von 0,001 % bis 0,01% kleiner als 1 ist, bevorzugt im Bereich von 0,4 bis 0,99 liegt, besonders bevorzugt im Bereich von 0,6 bis 0,95 liegt, und
- bei Auslenkungen im Bereich von 1% bis 10% größer als 1 ist, bevorzugt im Bereich von 1,01 bis 5 liegt, besonders bevorzugt im Bereich von 1,05 bis 3 liegt.

15. Dentale(r) Krone, Brücke, Prothesenzahn oder Vollprothese hergestellt durch 3D-Druck einer radikalisch polymerisierbaren Zusammensetzung nach einem der vorangehenden Ansprüche.

16. Radikalisch polymerisierbare Zusammensetzung nach einem der Ansprüche 1 bis 14 zur Anwendung in einem therapeutischen Verfahren, vorzugsweise in einem therapeutischen Verfahren als

- temporäre Krone oder Brücke,
- permanente Krone oder Brücke,
- Prothesenzahn,
- Prothesenbasis,
- Teilprothese oder
- Vollprothese.

Europäisches Patentamt
European Patent Office
Office européen des brevets

## EUROPÄISCHER RECHERCHENBERICHT

Nummer der Anmeldung

EP 24 15 5689

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 113 717 330 A (AIDITE QINHUANGDAO TECH CO LTD) 30. November 2021 (2021-11-30) * Absätze [0002] - [0004], [0008], [0020], [0025], [0061], [0065], [0072] * * Beispiele 1,3 * | 1-16 | INV. A61K6/17 A61K6/76 A61K6/77 A61K6/887 B33Y70/10 B33Y80/00 |
| E | -& EP 4 378 970 A1 (AIDITE QINHUANGDAO TECH CO LTD [CN]) 5. Juni 2024 (2024-06-05) * das ganze Dokument * | 1,2,4-7, 9-16 | |
| A | EVONIK INDUSTRIES: "AEROSIL fumed silica Product Finder - Full Text Search", INTERNET CITATION, 8. September 2010 (2010-09-08), Seiten 1-2, XP007914784, Gefunden im Internet: URL:http://www.aerosil.com/lpa-productfinder/page/productsbytext/faces/p... [gefunden am 2010-09-08] * das ganze Dokument * | 1-16 | |

RECHERCHIERTE SACHGEBIETE (IPC)

A61K
B33Y

Der vorliegende Recherchenbericht wurde für alle Patentansprüche erstellt

| Recherchenort | Abschlußdatum der Recherche | Prüfer |
|---|---|---|
| Den Haag | 9. Juli 2024 | Grave, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 24 15 5689

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-07-2024

| Im Recherchenbericht angeführtes Patentdokument | Datum der Veröffentlichung | Mitglied(er) der Patentfamilie | Datum der Veröffentlichung |
|---|---|---|---|
| CN 113717330 A | 30-11-2021 | CN 113717330 A | 30-11-2021 |
| | | EP 4378970 A1 | 05-06-2024 |
| | | WO 2023024724 A1 | 02-03-2023 |
| EP 4378970 A1 | 05-06-2024 | CN 113717330 A | 30-11-2021 |
| | | EP 4378970 A1 | 05-06-2024 |
| | | WO 2023024724 A1 | 02-03-2023 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014078537 A1
- WO 2017223084 A1
- WO 2019048963 A1
- WO 2017155692 A1
- WO 2015126862 A1
- WO 2015126666 A1
- WO 2018231583 A1
- EP 3795359 A1
- EP 1508834 A1
- WO 2014098956 A1
- WO 2001012679 A1
- EP 3669854 A1
- WO 2020177921 A1
- EP 3479171 A1
- WO 2020016282 A1
- DE 19616781 A1


**In der Beschreibung aufgeführte Nicht-Patentliteratur**

- *CHEMICAL ABSTRACTS,* 42405-01-6
- *CHEMICAL ABSTRACTS,* 42404-50-2
- *CHEMICAL ABSTRACTS,* 1834525-17-5
- *CHEMICAL ABSTRACTS,* 41637-38-1
- *CHEMICAL ABSTRACTS,* 72869-86-4
- *CHEMICAL ABSTRACTS,* 109-16-0
- *CHEMICAL ABSTRACTS,* 6701-13-9
- *CHEMICAL ABSTRACTS,* 7534-94-3
- *CHEMICAL ABSTRACTS,* 10595-06-9
- *CHEMICAL ABSTRACTS,* 999-97-3
- *CHEMICAL ABSTRACTS,* 2530-85-0
- *CHEMICAL ABSTRACTS,* 75980-60-8
- *CHEMICAL ABSTRACTS,* 128-37-0
- *CHEMICAL ABSTRACTS,* 84434-11-7